# EUROPÄISCHE PATENTSCHRIFT

(11) **EP 2 977 004 B1**
(45) Veröffentlichungstag und Bekanntmachung des Hinweises auf die Patenterteilung: **02.10.2019**
(21) Anmeldenummer: 15181075.1
(22) Anmeldetag: 11.04.2012
(51) Int. Cl.: A61B 5/1455

(54) **ANALYTISCHES HILFSMITTEL**
ANALYTICAL AID
MOYEN D'AIDE ANALYTIQUE

(30) Priorität: 12.04.2011 EP 11162068
(43) Veröffentlichungstag der Anmeldung: 27.01.2016
(62) Teilanmeldung aus: 12713981.4
(73) Patentinhaber: Roche Diabetes Care GmbH, 68305 Mannheim (DE); F. Hoffmann-La Roche AG, 4070 Basel (CH)
(72) Erfinder: LIST, Hans, 64754 Hesseneck-Kailbach (DE); SCHERER, Karl-Heinz, 68647 Biblis (DE)
(74) Vertreter: Altmann Stößel Dick Patentanwälte PartG mbB

(56) Entgegenhaltungen:
- EP-A1- 2 226 007
- EP-A2- 1 543 935
- US-A1- 2005 283 094

## Beschreibung

### Gebiet der Erfindung

Die Erfindung betrifft ein Verfahren zum Herstellen mehrerer analytischer Hilfsmittel zum Nachweis mindestens eines Analyten in einer Probe. Weiterhin betrifft die Erfindung ein analytisches Magazin, welches eine Mehrzahl derartiger analytischer Hilfsmittel umfasst. Derartige analytische Hilfsmittel werden allgemein insbesondere in der medizinischen Diagnostik eingesetzt, um einen oder mehrere Analyte qualitativ oder quantitativ in Proben, insbesondere flüssigen Proben und besonders bevorzugt in Körperflüssigkeiten, wie Blut, interstitielle Flüssigkeit, Speichel oder Urin, nachzuweisen. Beispielsweise kann es sich bei diesen Analyten um einen oder mehrere Metabolite handeln, beispielsweise Blutglucose.

### Stand der Technik

Im Bereich der Diagnostik ist es in vielen Fällen notwendig, Proben von Körperflüssigkeit, insbesondere Blutproben oder Proben interstitieller Flüssigkeit, zu gewinnen, um darin einen oder mehrere Analyte nachweisen zu können, insbesondere spezifisch. Beispiele derartiger Analyte, die auch im Rahmen der vorliegenden Erfindung nachgewiesen werden können, sind Glucose, insbesondere Blutglucose, Gerinnungsparameter, Triglyceride, Lactat, Cholesterin oder Kombinationen der genannten und/oder anderer Metabolite. Entsprechend der nachgewiesenen Konzentrationen kann dann beispielsweise über eine entsprechende Behandlung entschieden werden.

Zum Zweck des Analytnachweises werden in der Regel ein oder mehrere analytische Hilfsmittel eingesetzt, um die Proben zu gewinnen und/oder zu analysieren. So können die analytischen Hilfsmittel beispielsweise eine oder mehrere Lanzetten umfassen, also Elemente, welche eingerichtet sind, um eine Öffnung in einer Haut eines Benutzers zu erzeugen, durch welche die Körperflüssigkeit entnommen werden kann. Als Beispiel für derartige Lanzetten kann auf WO 01/36010 A1 verwiesen werden. Alternativ oder zusätzlich können die analytischen Hilfsmittel ein oder mehrere Testelemente mit einer oder mehreren Testchemien umfassen, welche eingerichtet sein können, um bei Einwirkung des nachzuweisenden Analyten bestimmte nachweisbare Eigenschaften zu ändern. Beispielsweise kann der Analytnachweis einen Nachweis elektrochemischer Eigenschaften der Analyte selbst und/oder anderer Stoffe umfassen und/oder eine Änderung elektrochemisch nachweisbarer Eigenschaften. Alternativ oder zusätzlich lassen sich auch beispielsweise optische Eigenschaften und/oder deren Änderungen nachweisen. Für die Beschreibung möglicher Testchemien kann beispielsweise auf J. Hönes et al.: Diabetes Technology and Therapeutics, Vol. 10, Supplement 1, 2008, S-10 bis S-26, verwiesen werden. Weiterhin kann beispielsweise auf WO 2010/094426 A1 oder auf WO 2010/094427 A1 verwiesen werden. Auch in diesen Dokumenten werden analytische Hilfsmittel mit einer Testchemie beschrieben, welche auch im Rahmen der vorliegenden Erfindung grundsätzlich einsetzbar ist.

Aus EP 1 543 934 A2 ist ein Kunststoff-Spritzgussteil mit eingebettetem Bauteil sowie ein entsprechendes Herstellungsverfahren bekannt. Beispielsweise beschreibt dieses Dokument die Herstellung von so genannten Analysechips, die mit einem Kunststoffrahmen umspritzt werden. Unter anderem wird in Absatz [0028] ein Verfahren beschrieben, bei welchem ein Glaskörper mit einem Kunststoffrahmen umgeben wird. Insbesondere bietet sich das Verfahren bei der Herstellung von so genannten Bio-Chips an. Bei diesen werden die unbehandelten Oberflächen des Glasträgers nachträglich, also nach dem Verbinden des Kunststoffrahmens mit dem Glasträger, mit entsprechenden Reagenzien beschichtet, die zum Einsatz von Bio-Chips erforderlich sind.

Weiterhin sind integrierte analytische Hilfsmittel bekannt, welche sowohl dem Zweck der Erzeugung der Probe der Körperflüssigkeit als auch dem Zweck eines Transports der Probe und gegebenenfalls sogar dem Zweck einer qualitativen und/oder quantitativen Analyse dieser Probe dienen. Beispiele derartiger analytischer Hilfsmittel sind Hilfsmittel, die so genannte Microsampler enthalten, bei denen mittels einer Lanzette ein Einstich oder ein Einschnitt erzeugt wird, bei denen die Probe aufgenommen wird und zu einem oder mehreren Testfeldern mit der Testchemie transportiert wird. Diese Testfelder können separat von der Lanzette angeordnet sein, können jedoch auch Bestandteil der Lanzette selbst sein. Derartige Systeme, welche beispielsweise in US 2004/0193202 A1, in US 2008/0249435 A1, in WO 03/009759 A1, in WO 2010/094427 A1 oder in WO 2010/094426 A1 beschrieben werden, sind aufgrund ihres hohen Integrationsgrades besonders benutzerfreundlich.

In EP 2 226 007 A1 wird ein analytisches Magazin mit Kammern beschrieben, in denen analytische Hilfsmittel gelagert sind. Das analytische Hilfsmittel umfasst mindestens ein Testelement zum Nachweis mindestens eines Analyten in einer Probe einer Körperflüssigkeit. Das Testelement umfasst ein Testfeld, wobei das Testfeld zumindest teilweise innerhalb der jeweiligen Kammer angeordnet ist und wobei eine Wand der Kammer das Testfeld zumindest teilweise überdeckt und eine von der Kammer aus zugängliche Testfeldfläche zumindest teilweise begrenzt. Beispielsweise wird in Absatz [0055] ausgeführt, dass ein Verbinden des Testfeldes mit dem Gehäuse auch zeitlich unabhängig und/oder prozessual unabhängig von dem Verbinden einer Membran mit dem Gehäuse erfolgen kann. Eine Aufbringung der Testfelder kann zu einem späteren Zeitpunkt erfolgen, so dass Halbfertigelemente ohne Testfelder zwischengelagert werden können.

In US 2005/0283094 A1 wird eine Testvorrichtung zum Testen einer Körperflüssigkeit beschrieben, mit einem Teststreifen und einem Stechelement sowie einem Gehäuse. Wie beispielsweise in Absatz [0052] beschrieben wird, können der Teststreifen und das Gehäuse auch einstückig ausgestaltet werden, so dass das Gehäuse oder ein Teil desselben auch als Teststreifen fungiert. Beispielsweise kann zur Erzeugung dieses einstückigen Testelement-Gehäuses ein Formgebungsverfahren eingesetzt werden.

Analytische Hilfsmittel werden in der Regel in Form von Magazinen bereitgestellt oder hergestellt, in welchen eine Mehrzahl derartiger analytischer Hilfsmittel aufgenommen ist. Diese analytischen Hilfsmittel, welche in der Regel als Einweg-Hilfsmittel ausgestaltet sind (Disposables) werden beispielsweise bei der Herstellung in einen ein- oder mehrteiligen Magazinkörper eingefügt. Dieser Vorgang des Einfügens ist jedoch in der Praxis vergleichsweise aufwendig, da in der Regel sehr kleine Strukturen mit sehr kleinen Disposables bestückt werden müssen. Insbesondere bei analytischen Hilfsmitteln in Form von Testelementen, die fest in ein Magazin integriert werden, ist dieser Vorgang aufwendig, da zunächst eine partielle Haftungseigenschaft des Magazins hergestellt werden muss, beispielsweise indem Klebstoff gezielt aufgedruckt wird oder indem doppelseitiges Klebeband als Zuschnitt eingefügt wird. Erst danach kann in der Regel die Montage eines eigentlichen Testfeldes, welches die Testchemie aufweist, erfolgen.

### Aufgabe der Erfindung

Es ist daher eine Aufgabe der vorliegenden Erfindung, ein analytisches Magazin sowie ein Verfahren zu Herstellung mehrerer analytischer Hilfsmittel bereit zu stellen, welche die Nachteile bekannter analytischer Hilfsmittel und Verfahren zumindest weitgehend vermeiden. Insbesondere soll das Verfahren kostengünstig realisierbar und großtechnisch durchführbar sein, wobei insbesondere ein Aufwand für eine Implementierung der Testchemie in das analytische Hilfsmittel gegenüber bekannten Herstellungsverfahren verringert werden soll.

### Offenbarung der Erfindung

Diese Aufgabe wird gelöst durch die Erfindung mit den Merkmalen der unabhängigen Ansprüche. Vorteilhafte Weiterbildungen der Erfindung, welche einzeln oder in Kombination realisierbar sind, sind in den abhängigen Ansprüchen dargestellt.

In einem ersten Aspekt der vorliegenden Beschreibung wird ein Verfahren zum Herstellen mehrerer analytischer Hilfsmittel zum Nachweis mindestens eines Analyten in einer Probe vorgeschlagen. Wie oben ausgeführt, ist im Rahmen der vorliegenden Erfindung unter einem analytischen Hilfsmittel allgemein eine Vorrichtung zu verstehen, welche eingerichtet ist, um mindestens einen Analyten qualitativ oder vorzugsweise quantitativ nachzuweisen, insbesondere spezifisch, und/oder welche bei einem derartigen Analytnachweis eingesetzt werden kann, beispielsweise zur Gewinnung der Probe und/oder zum Nachweis des Analyten in der Probe. Bei diesem mindestens einen Analyten kann es sich grundsätzlich um eine beliebige nachweisbare Substanz handeln. Besonders bevorzugt handelt es sich bei diesem Analyten um mindestens einen Metaboliten, insbesondere einen oder mehrere der eingangs genannten Metaboliten. Bei der Probe kann es sich insbesondere um eine Körperflüssigkeit handeln, beispielsweise, wie oben ausgeführt, um Blut, interstitielle Flüssigkeit, Speichel oder Urin. Das analytische Hilfsmittel kann eingerichtet sein, um den Nachweis des Analyten eigenständig durchzuführen, so dass beispielsweise unmittelbar ein Ergebnis des Nachweises bereitgestellt wird, oder in Zusammenwirkung mit einem Testgerät oder einer Testvorrichtung, welche das analytische Hilfsmittel verwendet. Das analytische Hilfsmittel kann insbesondere als Einweg-Hilfsmittel ausgestaltet sein, also für genau einen Test verwendbar sein. Das analytische Hilfsmittel istBestandteil eines analytischen Magazins oder ist in einem analytischen Magazin aufgenommen, wobei das analytische Magazin eine Mehrzahl analytischer Hilfsmittel umfasst, welche miteinander verbunden sind, beispielsweise starr miteinander verbunden sind und/oder einstückig ausgebildet sind. Beispielsweise kann ein analytisches Magazin realisiert werden, welches ein Magazingehäuse aufweist, wobei das analytische Magazin mehrere analytische Hilfsmittel umfasst, welche beispielsweise in dem Magazingehäuse aufgenommen sind und/oder welche über das Magazingehäuse miteinander verbunden sind. Beispielsweise können die Gehäuse der analytischen Hilfsmittel oder Teile derselben ganz oder teilweise Bestandteile des Magazingehäuses sein.

Das analytische Hilfsmittel gemäß der vorliegenden Beschreibung kann, im Gegensatz zur Erfindung, auch als Einzeltest ausgebildet sein. Unter einem Einzeltest wird dabei ein einzelnes analytisches Hilfsmittel verstanden, welches einzeln gehandhabt werden kann, unabhängig von weiteren analytischen Hilfsmitteln und ohne unmittelbare mechanische Verbindung mit weiteren analytischen Hilfsmitteln. Erfindungsgemäß sind jedoch mehrere analytische Hilfsmittel in einem Magazin aufgenommen und zu einem Magazin zusammengefasst, indem die analytischen Hilfsmittel durch ein gemeinsames Gehäuse mechanisch miteinander verbunden werden.

Das analytische Hilfsmittel umfasst mindestens ein Gehäuse und mindestens ein Testelement mit mindestens einer Testchemie. Unter einem Gehäuse ist allgemein ein Element zu verstehen, welches ein- oder mehrteilig ausgestaltet sein kann und welches eingerichtet ist, um das analytische Hilfsmittel zumindest weitgehend nach außen abzuschließen und/oder um einen mechanischen Schutz für das analytische Hilfsmittel bereitzustellen, insbesondere einen mechanischen Schutz gegenüber Einwirkungen von außen. Das Gehäuse kann dementsprechend vorzugsweise starr ausgebildet sein, so dass sich dieses beispielsweise unter Einwirkung üblicher, im Gebrauch auftretender mechanischer Kräfte, insbesondere unter Einwirkung seiner eigenen Gewichtskraft, nicht oder nur unwesentlich verformt. Beispielsweise kann das Gehäuse eine Gehäusewand aufweisen, welche in mindestens einem Bereich eine Wandstärke von mindestens 0,5 mm, vorzugsweise von mindestens 1,0 mm, aufweist. Alternativ oder zusätzlich sind jedoch grundsätzlich auch verformbare Gehäuse einsetzbar. Das Gehäuse kann insbesondere, wie unten noch näher ausgeführt wird, einen Innenraum aufweisen, welcher von dem Gehäuse oder Teilen des Gehäuses ganz oder teilweise umschlossen wird, beispielsweise mindestens eine Kammer. Diese Kammer kann beispielsweise eine oder mehrere Öffnungen aufweisen, durch welche hindurch beispielsweise ein Eingriff eines oder mehrerer Aktoren in die Kammer hinein möglich ist und/oder durch welches hindurch ein Transfer der Probe in die Kammer hinein möglich ist und/oder durch welche hindurch mindestens eine Eigenschaftsänderung des Testelements und/oder der Testchemie detektiert werden kann.

Besonders bevorzugt ist eine Ausgestaltung, bei welcher das analytische Hilfsmittel mindesten eine Kammer aufweist, also mindestens einen Innenraum oder Hohlraum, welcher von dem Gehäuse vollständig oder teilweise umschlossen wird. Beispielsweise kann die Kammer einen von dem Gehäuse umschlossenen Innenraum aufweisen, welcher lediglich durch eine oder mehrere Öffnungen zugänglich sein kann. Vorzugsweise ist das analytische Hilfsmittel derart ausgestaltet, dass mindestens eine Testfeldfläche der Kammer zuweist, so dass von der Kammer aus die Testfeldfläche des Testelements für eine Probenaufgabe auf die Testfeldfläche zugänglich ist.

Besonders bevorzugt kann das analytische Hilfsmittel, wie nachstehend weiterhin ausgeführt wird, weiterhin mindestens ein Lanzettenelement aufweisen. Dieses Lanzettenelement kann insbesondere vollständig oder teilweise in der mindestens einen Kammer aufgenommen sein, vorzugsweise beweglich. So kann das analytische Hilfsmittel beispielsweise genau eine Kammer aufweisen, mit genau einem Lanzettenelement, welches in der Kammer gelagert ist, vorzugsweise beweglich, so dass beispielsweise durch eine Öffnung in dem Gehäuse eine Stechbewegung durchführbar ist, bei welcher eine Lanzettenspitze des Lanzettenelements aus der Öffnung austritt. Vorzugsweise ist das Lanzettenelement nach der Stechbewegung in der Kammer remagazinierbar. Zusätzlich kann beispielsweise das Testelement mindestens eine Testfeldfläche aufweisen, die der Kammer zuweist.

Alternativ können auch beispielsweise mehrere analytische Hilfsmittel vorgesehen sein, mit jeweils einer oder mehreren Kammern, wobei die mehreren analytischen Hilfsmittel beispielsweise zu dem analytischen Magazin zusammengefasst sein können. Beispielsweise können das analytische Magazin und/oder die analytischen Hilfsmittel eine Mehrzahl von Kammern aufweisen, wobei in jeder Kammer vorzugsweise genau ein Lanzettenelement aufgenommen ist, vorzugsweise beweglich, so dass beispielsweise durch eine Öffnung in dem Gehäuse eine Stechbewegung durchführbar ist, bei welcher eine Lanzettenspitze des Lanzettenelements aus der Öffnung austritt. Auch in diesem Fall ist vorzugsweise pro Kammer mindestens eine Testfeldfläche des mindestens einen Testelements vorgesehen, welche der Kammer zuweist und welche von der Kammer aus mit Probe beaufschlagbar ist. So können beispielsweise in dem analytischen Magazin jeweils eine Kammer mit dem zugeordneten Lanzettenelement, optional der mindestens einen der Kammer zugeordneten Testfeldfläche und mit dem Gehäuse, welches die Kammer umschließt, ein analytisches Hilfsmittel bilden, wobei mehrere gleichartiger oder verschiedener analytischer Hilfsmittel zu dem analytischen Magazin zusammengefasst sind.

Unter einer Testchemie ist im Rahmen der vorliegenden Erfindung eine Substanz zu verstehen, welche eine oder mehrere chemische Komponenten umfassen kann, welche eingerichtet ist, um bei Anwesenheit des Analyten mindestens eine nachweisbare Eigenschaft zu ändern. Beispielsweise kann es sich bei dieser mindestens einen nachweisbaren Eigenschaft um mindestens eine elektrochemische Eigenschaft und/oder mindestens eine optische Eigenschaft handeln. Bezüglich möglicher Ausgestaltungen von Testchemien kann beispielsweise auf die obige Beschreibung des Standes der Technik verwiesen werden. Die Erfindung wird im Folgenden im Wesentlichen unter Bezugnahme auf Testchemien beschrieben, welche bei Anwesenheit des mindestens einen Analyten mindestens eine optisch nachweisbare Eigenschaft ändern, beispielsweise eine Farbe. Dementsprechend kann die Testchemie beispielsweise eingerichtet sein, um bei Anwesenheit des Analyten einen Farbumschlag durchzuführen. Grundsätzlich sind jedoch, alternativ oder zusätzlich, auch andere Arten von Testchemien einsetzbar, beispielsweise elektrochemische Testchemien.

Unter einem Testelement wird im Rahmen der vorliegenden Erfindung ein ein- oder mehrteiliges, vorzugsweise ein einteiliges, Element verstanden, welches die mindestens eine Testchemie aufweist. Wie unten noch näher ausgeführt wird, kann das Testelement beispielsweise mindestens ein Trägerelement umfassen, in welches die Testchemie eingebracht ist und/oder auf welches die Testchemie aufgebracht ist. Insbesondere kann das mindestens eine Trägerelement von dem Gehäuse getrennt hergestellt und/oder bereitgestellt werden. Beispielsweise kann das mindestens eine Trägerelement mindestens ein Kunststoffmaterial umfassen, wobei jedoch grundsätzlich auch andere Materialien alternativ oder zusätzlich einsetzbar sind, beispielsweise keramische Materialien und/oder Papiermaterialien und/oder Glasmaterialien. Auch Verbundmaterialien sind grundsätzlich einsetzbar. Unter einem Trägerelement wird allgemein ein Element verstanden, welches eingerichtet ist, um die Testchemie zu tragen, beispielsweise indem die Testchemie auf das Trägerelement in Form einer oder mehrerer Schichten aufgebracht ist. Alternativ oder zusätzlich kann das Trägerelement jedoch auch beispielsweise als Matrixmaterial dienen oder ein Matrixmaterial umfassen, in welches die Testchemie eingebracht ist. Wiederum alternativ oder zusätzlich kann das Trägerelement auch eine oder mehrere Aufnahmen umfassen, beispielsweise eine oder mehrere Vertiefungen, in welche die Testchemie eingebracht sein kann. Verschiedene Ausgestaltungen sind möglich.

Im Unterschied zum oben genannten Stand der Technik, insbesondere zur EP 1 543 934 A2, wird bei dem erfindungsgemäß vorgeschlagenen Verfahren das Testelement (welches am Ende des Verfahrens die Testchemie aufweist) in dem Formgebungsverfahren nicht nur mit dem Gehäuseteil verbunden, es werden auch mehrere Gehäuseteile gleichzeitig hergestellt und gleichzeitig miteinander verbunden. Auf diese Weise werden mehrere analytische Hilfsmittel in einem Magazin aufgenommen. Insbesondere kann dies derart erfolgen, dass das Trägerelement mit der darauf aufgebrachten Testchemie in das Formgebungsverfahren eingebracht wird und dort mit dem Gehäuseteil verbunden wird, beispielsweise indem das Testelement mit dem Trägerelement und der darauf aufgebrachten Testchemie in ein Werkzeug des Formgebungsverfahrens eingebracht und dort mit mindestens einem Gehäusewerkstoff des Gehäuseteils kontaktiert wird. Dabei können das Trägerelement und/oder die Testchemie mit dem Gehäusewerkstoff des Gehäuseteils kontaktiert werden. Beispielsweise kann das Trägerelement mit der darauf aufgebrachten Testchemie ganz oder teilweise mit dem Gehäusewerkstoff hinterspritzt werden und/oder teilweise in den Gehäusewerkstoff eingebettet werden. Hierdurch lässt sich eine erhebliche Vereinfachung des Verfahrens erzielen. Im Gegensatz hierzu wird beispielsweise in EP 1 543 934 A2 eine Oberfläche des Glasträgers nachträglich, also nach dem Verbinden des Glasträgers mit dem Kunststoffrahmen, mit entsprechenden Reagenzien beschichtet. Ein derartiger Schritt setzt jedoch eine aufwändige Reinigung der Glasoberfläche voraus. Zudem ist insbesondere bei kleinvolumigen analytischen Hilfsmitteln eine derartige nachträgliche Beschichtung mit Testchemie kaum mehr möglich, da die zu beschichtenden Flächen in der Regel nicht oder nur schwer zugänglich sind und in der Regel derart klein sind, dass eine sehr aufwändige und hoch präzise Aufbringung der Testchemie erforderlich wäre. Dieser erhebliche Aufwand des Standes der Technik lässt sich durch das vorgeschlagene Verfahren vermeiden.

Gleichzeitig kann das Testelement separat hergestellt werden und erst anschließend dem Formgebungsverfahren zugeführt werden. Damit unterscheidet sich das vorgeschlagene Verfahren beispielsweise von dem in EP 2 226 007 A1 beschriebenen Verfahren, bei welchem das eigentliche Testelement erst bei dem Formgebungsprozess entsteht. Das vorgeschlagene Verfahren bietet demgegenüber den Vorteil, dass beispielsweise ein Trägerelement des Testelements nicht gleichzeitig hinsichtlich seiner mechanischen Eigenschaften für den Einsatz als Gehäusematerial optimiert werden muss. Das Testelement lässt sich hinsichtlich seiner Fertigung und/oder hinsichtlich seiner Komponenten separat herstellen und optimieren.

Besonders bevorzugt ist es, wenn das Trägerelement ganz oder teilweise als Folienelement ausgestaltet ist oder ein derartiges Folienelement umfasst. Unter einem Folienelement ist dabei ein Element zu verstehen, dessen laterale Ausdehnung seine Dicke um mindestens einen Faktor 10, vorzugsweise um mindestens einen Faktor 100 und besonders bevorzugt um mindestens einen Faktor 1000 übersteigt. Beispielsweise kann sich das Folienelement unter Einwirkung seiner eigenen Gewichtskraft senkrecht zu seiner lateralen Ausdehnung verformen. Das Folienelement kann insbesondere als ebenes Folienelement ausgestaltet sein, mit einer Dicke von weniger als 1 mm, vorzugsweise mit einer Dicke von maximal 500 Mikrometern. Beispielsweise kann das Folienelement eine Dicke von 50 Mikrometern bis 1 mm aufweisen, insbesondere eine Dicke von 100 Mikrometern bis 500 Mikrometern und besonders bevorzugt eine Dicke von 140 Mikrometern bis 250 Mikrometern. Das Folienelement kann insbesondere flexibel oder verformbar ausgestaltet sein. Besonders bevorzugt ist es, wenn das Folienelement eine ringförmige Gestalt aufweist, beispielsweise eine Form eines Kreisrings.

Dar Trägerelement kann insbesondere ganz oder teilweise als optisch transparentes Trägerelement ausgestaltet sein. Unter einem optisch transparenten Trägerelement ist allgemein ein Element zu verstehen, welches eine Transparenz für optische Signale im ultravioletten und/oder sichtbaren und/oder infraroten Spektralbereich aufweist. Beispielsweise kann eine Transparenz für mindestens eine Wellenlänge in einem Wellenlängenbereich zwischen 200 nm und 400 nm bestehen und/oder eine Transparenz für mindestens eine Wellenlänge in einem Wellenlängenbereich zwischen 400 nm und 800 nm und/oder eine Transparenz für mindestens eine Wellenlänge in einem Wellenlängenbereich zwischen 800 nm und 1600 nm. Unter einer Transparenz ist dabei eine Eigenschaft zu verstehen, bei welcher Signale der genannten Wellenlängen nach Durchtritt durch das Trägerelement, beispielsweise nach Durchtritt senkrecht zu einer Oberfläche des Folienelements, eine Intensität aufweisen, welche mindestens 10 %, vorzugsweise mindestens 30 % und besonders bevorzugt mindestens 60 % einer Intensität vor Durchtritt durch das Trägerelement aufweisen.

Das vorgeschlagene Verfahren zur Herstellung des analytischen Hilfsmittels umfasst die nachfolgend beschriebenen Schritte. Diese Schritte können vorzugsweise in der dargestellten Reihenfolge durchgeführt werden. Weiterhin können jedoch auch einzelne der mehrere dieser Schritte in einer anderen Reihenfolge, zeitlich überlappend oder zeitlich parallel durchgeführt werden. Weiterhin können einzelne oder mehrere dieser Schritte wiederholt durchgeführt werden. Weiterhin können zusätzliche, im Folgenden nicht dargestellte Verfahrensschritte durchgeführt werden.

Die Verfahrensschritte sind:
a) Bereitstellen des Testelements; und
b) Herstellen mindestens eines Gehäuseteils des Gehäuses mittels mindestens eines Formgebungsverfahrens, wobei während des Formgebungsverfahrens das Testelement mit dem Gehäuseteil verbunden wird.

Unter einer Bereitstellung des Testelements kann beispielsweise eine Herstellung des Testelements verstanden werden. Alternativ oder zusätzlich kann eine Bereitstellung jedoch auch allgemein eine beliebige Art der Einführung des Testelements in das Verfahren beinhalten, welche dergestalt ist, dass das Testelement in das analytische Hilfsmittel implementierbar ist. Beispielsweise können analytische Hilfsmittel auch von einem anderen Hersteller und/oder in einem separaten Herstellungsbetrieb hergestellt und bereitgestellt werden und/oder in einer unabhängigen Produktionsanlage hergestellt werden. Es werden mehrere Testelemente gleichzeitig bereitgestellt, beispielsweise sämtliche Testelemente für alle analytischen Hilfsmittel eines analytischen Magazins und/oder ein Testelement für mehrere analytische Hilfsmittel. Die Bereitstellung kann in einem Einzelverfahren erfolgen oder auch in einem kontinuierlichen oder diskontinuierlichen Bereitstellungsverfahren für mehrere analytische Hilfsmittel, beispielsweise in Form von Bandware, wobei ein Band beispielsweise mehrere Testelemente umfasst, die nacheinander an einer Applikationsstelle bereitgestellt werden können.

Unter einem Gehäuseteil des Gehäuses ist im Rahmen der vorliegenden Erfindung eine Komponente des Gehäuses zu verstehen, welche einzeln oder nach Kombination mit einem oder mehreren weiteren Gehäuseteilen das Gehäuse gemäß der obigen Definition bildet. Insbesondere ist es möglich, das Gehäuse aus mehreren Gehäuseteilen in Form von kraftschlüssig und/oder stoffschlüssig und/oder formschlüssig miteinander verbundenen Gehäuseteilen auszubilden, beispielsweise in Form einer Gehäusebasis als erstes Gehäuseteil und in Form eines Gehäusedeckels als zweites Gehäuseteil und/oder in Form einer Oberschale als erstes Gehäuseteils und in Form einer Unterschale als zweites Gehäuseteil.

Unter einem Formgebungsverfahren wird im Rahmen der vorliegenden Erfindung ein Verfahren verstanden, bei welchem mindestens ein Gehäusewerkstoff und/oder mindestens ein Ausgangswerkstoff, aus welchem mindestens ein Gehäusewerkstoff entsteht, derart geformt wird, dass das Gehäuseteil entsteht. Beispielsweise können dieser Gehäusewerkstoff und/oder dieser mindestens eine Ausgangswerkstoff mindestens ein Kunststoffmaterial und/oder mindestens einen Ausgangswerkstoff für ein Kunststoffmaterial umfassen. In Letzterem Fall kann beispielsweise mindestens ein polymerisierbarer und/oder vernetzbarer und/oder aushärtbarer Ausgangswerkstoff oder eine Kombination mehrerer derartiger Ausgangswerkstoffe verwendet werden. So können beispielsweise ein oder mehrere Reaktionsharze und/oder ein oder mehrere lichtaushärtende und/oder thermisch aushärtbare Harze, beispielsweise ein oder mehrere Epoxidharze, verwendet werden. Alternativ oder zusätzlich können, wie unten noch näher ausgeführt wird, auch beispielsweise ein oder mehrere thermoplastische Kunststoffe und/oder ein oder mehrere duroplastische Kunststoffe und/oder ein oder mehrere elastomere Kunststoffe verwendet werden.

Beispielsweise kann das Formgebungsverfahren mindestens ein Gießverfahren umfassen. Dieses Gießverfahren kann beispielsweise ein Verfahren umfassen, bei welchem ein oder mehrere Harze, insbesondere Gießharze, in das Formnest eingebracht werden. Alternativ oder zusätzlich kann das Gießverfahren jedoch auch mindestens ein thermisches Gießverfahren umfassen, insbesondere ein Spritzgießverfahren.

Beispielsweise kann das Formgebungsverfahren mindestens ein Gießverfahren umfassen, insbesondere ein Gießverfahren, bei welchem mindestens ein Gehäusewerkstoff und/oder mindestens ein Ausgangswerkstoff für einen Gehäusewerkstoff durch einen Gießprozess in mindestens ein Formnest eingebracht werden. Unter einem Gießverfahren ist allgemein ein Verfahren zu verstehen, bei welchem mindestens ein Gehäusewerkstoff und/oder mindestens ein Ausgangswerkstoff für einen Gehäusewerkstoff in flüssiger, zähflüssiger oder verformbarer Form, was optional auch die Möglichkeit einer Verwendung mindestens eines Gehäusewerkstoffs mit thixotropen Eigenschaften und/oder mindestens eines thixotropen Ausgangswerkstoffs für einen Gehäusewerkstoff einschließt, in mindestens ein Formnest eingebracht wird. Beispielsweise kann der Gehäusewerkstoff als Schmelze in das Formnest eingebracht werden. Der Gießprozess kann unter Unterdruck, unter Normaldruck oder auch unter Überdruck erfolgen.

Beispielsweise kann der Gießprozess auch mindestens einen Spritzprozess umfassen, insbesondere einen thermischen Spritzprozess, beispielsweise einen Spritzgießprozess. Unter einem Spritzgießprozess ist allgemein im Rahmen der vorliegenden Erfindung ein Verfahren zu verstehen, bei welchem mindestens eine Schmelze mindestens eines Gehäusewerkstoffs in mindestens ein Formnest eingebracht wird, beispielsweise mindestens eine Kunststoffschmelze. Beispielsweise kann dabei eine Spritzgießmaschine verwendet werden, in welcher der mindestens eine Gehäusewerkstoff in mindestens einer Spritzeinheit plastifiziert wird und anschließend in mindestens ein Formnest mindestens eines Spritzgießwerkzeugs eingespritzt wird.

Der Gehäusewerkstoff und/oder der Ausgangswerkstoff können beispielsweise unter einem Druck von mindestens 2 bar, vorzugsweise von mindestens 10 bar, insbesondere sogar mindestens 100 bar, mindestens 500 bar oder sogar mindestens 1000 bar, in das Formnest des Werkzeuges eingebracht werden.

Wie unten noch näher ausgeführt wird, kann das Formgebungsverfahren insbesondere mindestens ein Kunststoff-Formgebungsverfahren umfassen und vorzugsweise ein Thermoforming-Verfahren also ein Verfahren, bei welchem der Gehäusewerkstoff bei erhöhter Temperatur, beispielsweise einer Temperatur von mindestens 50 °C, insbesondere mindestens 60 °C, vorzugsweise mindestens 80° C und besonders bevorzugt von mindestens 100° C, geformt wird. Dies kann beispielsweise im Rahmen eines Spritzgießverfahrens erfolgen. Das Formgebungsverfahren kann beispielsweise unter Verwendung mindestens eines Formgebungswerkzeugs erfolgen, beispielsweis unter Verwendung eines Werkzeugs, welches mindestens ein Formnest aufweist, in welchem das Gehäuseteil vollständig oder teilweise geformt wird. Das Formgebungsverfahren kann insbesondere derart ausgestaltet sein, dass nach Durchführung des Formgebungsverfahrens das Gehäuseteil bereits seine endgültige Form einnimmt, also insbesondere seine Form, welches das Gehäuseteil später in dem Gehäuse des analytischen Hilfsmittels einnimmt. Das Kunststoff-Formgebungsverfahren kann insbesondere ausgewählt sein aus der Gruppe bestehend aus einem Hinterspritzen und einem Umspritzen. Auch andere Kunststoff-Formgebungsverfahren sind jedoch alternativ oder zusätzlich einsetzbar.

Wie oben ausgeführt, wird während des Formgebungsverfahrens des Gehäuseteils das Testelement mit dem Gehäuseteil verbunden. Dies bedeutet, dass das Verbinden des Testelements gleichzeitig zu dem Formgebungsverfahren erfolgt und/oder zeitlich überlappend zu dem Formgebungsverfahren erfolgt. Zudem wird das Testelement durch das Formgebungsverfahren, also mittels und/oder aufgrund des Formgebungsverfahrens, mit dem Gehäuseteil verbunden, so dass der Vorgang des Verbindens des Testelements mit dem Gehäuseteil und der Vorgang des Formgebungsverfahrens zumindest teilweise identisch sind.

Unter einer Verbindung des Testelements mit dem Gehäuseteil kann grundsätzlich ein beliebiger Vorgang verstanden werden, nach dessen Durchführung das Testelement und/oder mindestens ein Teil des Testelements kraftschlüssig und/oder formschlüssig und/oder stoffschlüssig mit dem Gehäuseteil verbunden ist, so dass vorzugsweise das Testelement nicht mehr relativ zu dem Gehäuseteil bewegbar ist oder beispielsweise lediglich mit einer Toleranz, welche vorzugsweise in keiner Richtung mehr als 1 mm beträgt, vorzugsweise nicht mehr als 0,5 mm und besonders bevorzugt nicht mehr als 0,2 mm oder sogar nicht mehr als 0,1 mm oder sogar ohne jegliches Spiel. Bei der Verbindung kann es sich insbesondere um eine direkte Verbindung handeln, also eine Verbindung ohne mittelbares Verbindungselement, wie beispielsweise einen Klebstoff. Erfindungsgemäß wird das Testelement mittels des Formgebungsverfahrens insbesondere direkt mit dem Gehäuseteil verbunden, ohne Verwendung eines oder mehrerer mittelbarer Verbindungselemente wie beispielsweise Klebstoffen.

Insbesondere kann das Testelement derart mit dem Gehäuseteil verbunden werden, dass mindestens ein Bereich des Testelements, beispielsweise mindestens eine Fläche, das Gehäuseteil kontaktiert. Das Verfahren kann insbesondere derart durchgeführt werden, dass während des Formgebungsverfahrens das Testelement derart mit dem Gehäuseteil verbunden wird, dass das Testelement an mindestens einer Fläche auf dem Gehäuseteil aufliegt. Diese Fläche kann auch als Verbindungsfläche bezeichnet werden. Dieses Aufliegen sollte direkt erfolgen, also ohne Zwischenschaltung eines oder mehrerer Verbindungselemente wie beispielsweise Klebstoffen. Das Aufliegen kann beispielsweise auf genau einer Seite des Testelements erfolgen. Beispielsweise kann das Testelement während des Formgebungsverfahrens derart mit dem Gehäuseteil verbunden werden, dass dieses mit einer ersten Seite, beispielsweise mit einer ersten Oberfläche eines Folienelements, auf dem Gehäuseteil aufliegt, wobei beispielsweise eine zweite Oberfläche, beispielsweise eine gegenüberliegende Oberfläche des Folienelements, nicht aufliegend ausgestaltet sein kann. Alternativ oder zusätzlich kann das Verfahren jedoch auch derart durchgeführt werden, dass während des Formgebungsverfahrens das Testelement zumindest teilweise in das Gehäuseteil eingebettet wird. Dies bedeutet, dass das Testelement oder Teile desselben, beispielsweise ein oder mehrere Teile des Trägerelements, in mindestens zwei Dimensionen, vorzugsweise in mindestens drei Dimensionen vollständig oder teilweise von mindestens einem Gehäusewerkstoff des Gehäuseteils umschlossen sind, beispielsweise derart, dass ein direkter Kontakt zwischen dem Gehäusewerkstoff und dem Testelement besteht.

Wie oben ausgeführt, kann das Testelement insbesondere mit dem Gehäuseteil in einer Weise verbunden werden, ausgewählt aus der Gruppe bestehend aus einer stoffschlüssigen Verbindung, einer formschlüssigen Verbindung und einer kraftschlüssigen Verbindung. Auch Kombinationen der genannten Verbindungsarten sind möglich. Besonders bevorzugt sind eine formschlüssige und/oder eine kraftschlüssige Verbindung, insbesondere direkt, so dass ein unmittelbarer Kontakt zwischen dem Testelement und dem Gehäuseteil besteht.

Umfasst das Gehäuse mehrere Gehäuseteile, so betrifft der oben beschriebene Verfahrensschritt ein b) mindestens eins dieser Gehäuseteile, so dass beispielsweise das Gehäuse auch ein oder mehrere Gehäuseteile aufweisen kann, mit denen das Testelement nicht verbunden wird.

Wie oben beschrieben, kann das Formgebungsverfahren insbesondere mindestens ein Kunststoff-Formgebungsverfahren umfassen. Unter einem Kunststoff-Formgebungsverfahren wird ein Verfahren verstanden, bei welchem mindestens ein Gehäusewerkstoff in Form mindestens eines Kunststoffs und/oder mindestens eines Rohmaterials eines Kunststoffs einem Formgebungsverfahren unterworfen wird. Bei dem Kunststoff kann es sich insbesondere um einen thermoplastischen Kunststoff handeln, beispielsweise einen thermoplastischen Kunststoff, welcher bereits vor dem Formgebungsverfahren chemisch in seiner endgültigen Form vorliegt und welcher durch das Formgebungsverfahren lediglich in seiner äußeren Form umgestaltet wird. Auch andere Kunststoffe sind jedoch einsetzbar. Beispielsweise können ein oder mehrere Rohmaterialien eines Kunststoffs verwendet werden, welche erst bei dem Formgebungsverfahren einen Kunststoff bilden, beispielsweise durch eine chemische Reaktion, insbesondere eine Polymerisierung und/oder eine Vernetzung.

Insbesondere kann das Kunststoff-Formgebungsverfahren mindestens ein Thermoforming-Verfahren sein oder mindestens ein Thermoforming-Verfahren umfassen. Unter einem Thermoforming-Verfahren ist, wie oben ausgeführt, ein Formgebungsverfahren zu verstehen, bei welchem ein Wärmeeintrag in mindestens ein Gehäusewerkstoff erfolgt. Dies kann beispielsweise unter Verwendung mindestens eines beheizten Werkzeugs erfolgen, beispielsweise unter Verwendung mindestens eines beheizten Werkzeugs mit mindestens einem Formnest. Das Kunststoff-Formgebungsverfahren kann insbesondere ausgewählt sein aus der Gruppe bestehend aus: einem Gießverfahren, insbesondere einem Spritzgießverfahren; einem Pressverfahren, insbesondere einem Spritzpressverfahren. Auch Kombinationen der genannten Verfahren und/oder eine Verwendung eines oder mehrerer anderer Kunststoff-Formgebungsverfahren sind jedoch grundsätzlich möglich.

Besonders bevorzugt ist es, wenn das Kunststoff-Formgebungsverfahren ausgewählt ist aus der Gruppe bestehend aus einem Hinterspritzen und einem Umspritzen, wobei auch Kombinationen dieser Techniken möglich sind. Damit kann das Verbindungsverfahren des Testelements mit dem Gehäuseteil insbesondere ein Hinterspritzen oder ein Umspritzen umfassen, beispielsweise ein Folienhinterspritzen und/oder ein Folienumspritzen. Bei einem Hinterspritzen und einem Umspritzen wird das Testelement zunächst zumindest teilweise in ein Werkzeug eingelegt, beispielsweise in ein Formnest eines Werkzeugs, so dass ein Teil des Formnests frei bleibt. Dieser Teil wird anschließend beispielsweise mit einem oder mehreren Kunststoffmaterialien und/oder mit einem oder mehreren Rohmaterialien eines Kunststoffinaterials gefüllt, beispielsweise in Form eines Gießprozesses, eins Spritzprozesse oder eines Pressprozesses. Bei einem Hinterspritzen wird dabei das Einlegen des Testelements in das Formnest derart ausgeführt, dass eine Seite des Testelements mit dem Kunststoffmaterial in direkten Kontakt gerät. Bei einem Umspritzen erfolgt das Einlegen derart, dass das Testelement zumindest teilweise in mindestens zwei, insbesondere in drei Dimensionen von dem Kunststoffwerkstoff umschlossen wird.

So kann allgemein in dem oben beschriebenen Verfahrensschritt b) mindestens ein Teil des Testelements in ein Formgebungswerkzeug eingelegt werden und in dem Formgebungswerkzeug zumindest teilweise mit einem Gehäusewerkstoff des Gehäuseteils, beispielsweise einem Kunststoffwerkstoff, in Kontakt gebracht werden. Dieses Inkontaktbringen kann einseitig erfolgen.

Bei einem Folienelement kann dieses Inkontaktbringen beispielsweise auf einer Folienseite erfolgen. Alternativ oder zusätzlich kann das Inkontaktbringen jedoch auch in zwei oder mehr Dimensionen erfolgen, so dass das Testelement beispielsweise bei dem Umhüllen zumindest teilweise in zwei oder drei Dimensionen von dem Gehäusewerkstoff umschlossen wird. In beiden Fällen sollte vorzugsweise ein direkter Kontakt zwischen dem Gehäusewerkstoff und dem Testelement oder Teilen desselben bestehen.

Wird ein Formgebungswerkzeug verwendet, so ist es besonders bevorzugt, wenn der mindestens eine in das Formgebungswerkzeug eingelegte Teil des Testelements mit mindestens einem Abschnitt auf mindestens einer Wand des Formgebungswerkzeugs aufliegt. Auf diese Weise kann beispielsweise sichergestellt werden, dass dieser Abschnitt, welcher auf der Wand des Formgebungswerkzeugs aufliegt, nicht mit dem Gehäusewerkstoff bedeckt wird. Alternativ oder zusätzlich kann dieser Abschnitt, welcher auf der Wand des Formgebungswerkzeugs aufliegt, beispielsweise gesondert temperiert werden.

So kann beispielsweise der Verfahrensschritt b) derart durchgeführt werden, dass die Wand des Formgebungswerkzeugs, zumindest in dem Bereich, auf welchem der Abschnitt des Testelements auf der Wand aufliegt, während der Durchführung des Verfahrensschritts b) eine Temperatur von nicht mehr als 130° C, bis vorzugsweise von nicht mehr als 120° C und besonders bevorzugt von nicht mehr als 110° C aufweist. Dies kann beispielsweise durch eine entsprechende Temperierung des Werkzeugs erfolgen. Beispielsweise kann diese Temperaturobergrenze bei Verwendung eines Gießverfahrens, insbesondere eines Spritzgießverfahrens, vorliegen. Weiterhin kann, insbesondere bei Verwendung eines thermischen Formgebungsverfahrens und besonders bevorzugt eines Spritzgießverfahrens, beispielsweise der Verfahrensschritt b) derart durchgeführt werden, dass die Wand des Formgebungswerkzeugs, zumindest in dem Bereich, auf welchem der Abschnitt des Testelements auf der Wand aufliegt, während der Durchführung des Verfahrensschritts b) eine Temperatur von mindestens 25 °C aufweist, insbesondere mindestens 30 °C, vorzugsweise mindestens 40 °C und besonders bevorzugt mindestens 50 °C, beispielsweise durch entsprechende Temperierung des Formgebungswerkzeugs. Beispielsweise kann der Verfahrensschritt b) derart durchgeführt werden, insbesondere bei Verwendung eines thermischen Formgebungsverfahrens und besonders bevorzugt eines Spritzgießverfahrens, dass die Wand des Formgebungswerkzeugs, zumindest in dem Bereich, auf welchem der Abschnitt des Testelements auf der Wand aufliegt, während der Durchführung des Verfahrensschritts b) eine Temperatur von 30 °C bis 130 °C aufweist, insbesondere von 40 °C bis 120 °C und besonders bevorzugt von 50 °C bis 110 °C, beispielsweise durch entsprechende Temperierung des Formgebungswerkzeugs. Derartige Temperierungen können beispielsweise durch so genannte FEM-Simulationen, welche routinemäßig bei der Werkzeuggestaltung in vielen Fällen durchgeführt werden, und/oder durch andere Simulationen und/oder empirische Verfahren, bestimmt werden. So kann durch ein geeignetes Werkzeugdesign und/oder durch einen geeigneten Betrieb des Werkzeugs sichergestellt werden, dass die oben genannten Temperaturen aufrecht erhalten werden.

Der mindestens eine Abschnitt des Testelements, welcher auf der Wand aufliegt, kann beispielsweise eine Seite eines streifenförmigen und/oder scheibenförmigen Testelements sein. Insbesondere kann der Abschnitt des Testelements, welcher auf der Wand aufliegt, zumindest einen Teil der Testchemie umfassen, insbesondere eine Testfeldfläche der Testchemie. So kann beispielsweise sichergestellt werden, dass die Testchemie nicht oder zumindest nicht vollständig mit dem Gehäusewerkstoff bedeckt wird. Weiterhin kann sichergestellt werden, dass die Testchemie während des Formgebungsverfahrens lediglich einer Temperatur ausgesetzt wird, bei welcher diese, zumindest kurzfristig, noch keine Schädigung erfährt.

Das analytische Hilfsmittel kann insbesondere mindestens eine Kammer umfassen, also einen Hohlraum, welche vollständig oder teilweise von dem Gehäusewerkstoff des Gehäuses umgeben und/oder begrenzt ist. Das Testelement kann insbesondere eine Testfeldfläche aufweisen, in welcher die Testchemie dem Innenraum der Kammer zuweist und somit vorzugsweise von der Kammer aus für eine Aufgabe der Probe zugänglich ist. Auch derartige komplexe Strukturen mit im Inneren einer Kammer angeordneten Testfeldflächen sind mit dem erfindungsgemäßen Verfahren problemlos realisierbar, da, im Unterschied zum Stand der Technik, eine nachträgliche Beschichtung der Testfeldflächen mit Testchemie nach dem Erzeugen des Gehäuses nicht erforderlich ist. Eine derartige Struktur wäre beispielsweise mit dem in EP 1 543 934 A2 beschriebenen Verfahren nicht realisierbar.

Das Formgebungsverfahren kann allgemein insbesondere Trennmittel-frei durchgeführt werden. Dies bedeutet, dass vorzugsweise auf einer Wand des Formnests, welche mit dem Gehäusewerkstoff und/oder dem Testelement in Kontakt gerät, vorzugsweise kein Trennmittel aufgebracht wird, so dass das Gehäusewerkstoff und/oder das Testelement vorzugsweise direkt auf der Wand des Formnests des Formgebungswerkzeugs aufliegen.

Das Verfahren kann weiterhin insbesondere derart durchgeführt werden, dass im Verfahrensschritt b) das Testelement derart mit dem Gehäuseteil verbunden wird, dass mindestens eine Oberfläche der Testchemie als Testfeldfläche für eine Aufgabe der Probe von einem Gehäusewerkstoff des Gehäuseteils unbedeckt verbleibt. Unter einer Testfeldfläche ist somit eine Oberfläche der Testchemie zu verstehen, welche für den Nachweis des Analyten eingesetzt werden kann und welche mit der Probe oder einem Bestandteil der Probe in Kontakt geraten kann, beispielsweise indem die Probe senkrecht zur Testfeldfläche oder parallel zur Testfeldfläche auf die Testfeldfläche aufgebracht wird. Dieses Aufbringen kann beispielsweise mittels eines Transferelements und/oder direkt erfolgen.

Diese unbedeckte Ausgestaltung der Testfeldfläche, wobei auch mehrere Testfeldflächen vorgesehen sein können, kann auf verschiedene Weisen erfolgen. So kann beispielsweise das Testelement, wie oben beschrieben, derart hinterspritzt werden, dass die Testfeldfläche auf einer dem Gehäuseteil gegenüberliegenden Seite des Trägerelements angeordnet ist, so dass beispielsweise auf einer ersten Seite das Trägerelement mit dem Gehäuseteil in Kontakt steht, und das auf einer gegenüberliegenden zweiten Seite die Testchemie auf das Trägerelement aufgebracht ist. Auch andere Ausgestaltungen sind jedoch grundsätzlich möglich. Alternativ oder zusätzlich kann die unbedeckte Ausgestaltung der Testfeldfläche auch derart erfolgen, dass das Trägerelement zwar auf der Seite der Testchemie teilweise mit dem Gehäusewerkstoff bedeckt wird oder mit dem Gehäusewerkstoff in Kontakt gerät, wobei jedoch die Testfeldfläche unbedeckt verbleibt, beispielsweise indem das Gehäuseteil auf der Seite der Testfeldfläche ein oder mehrere Aufgabefenster bildet, durch welche hindurch die Testfeldfläche für die Probenaufgabe zugänglich ist, wobei die Aufgabefenster beispielsweise die Testfeldfläche vollständig oder teilweise umrahmen. Die Testfeldfläche kann von einer Außenseite des Gehäuses zugänglich sein oder kann, wie unten noch näher ausgeführt wird, insbesondere im Inneren einer Kammer angeordnet sein, so dass beispielsweise die Probenaufgabe vom Innenraum der Kammer aus erfolgen kann.

Das Verfahren kann weiterhin insbesondere derart durchgeführt werden, dass das Gehäuse derart ausgestaltet wird, dass mindestens ein Sichtfenster in dem Gehäuse entsteht, wobei die Testchemie, insbesondere die Testfeldfläche, durch das Sichtfenster hindurch optisch überwacht werden kann, beispielsweise von außen. So kann insbesondere mindestens ein Sichtfenster in dem Gehäuse gebildet werden, beispielsweise ein oder mehrere Sichtfenster pro analytischem Hilfsmittel, wobei die Testfeldfläche durch das Sichtfenster hindurch optisch überwachbar ist. Diese Überwachung kann direkt oder indirekt erfolgen, beispielsweise indem durch das Sichtfenster in dem Gehäuse hindurch ein direkter Blick auf die Testfeldfläche und/oder die Testchemie ermöglicht wird. Alternativ oder zusätzlich kann die optische Überwachung jedoch auch durch ein oder mehrere optisch transparente Elemente hindurch erfolgen, wobei bezüglich der optischen Transparenz auf die obige Definition verwiesen werden kann. Beispielsweise kann dieses optisch transparente Element Bestandteil des Gehäuses sein. Alternativ oder zusätzlich kann das optisch transparente Element jedoch auch das optionale Trägerelement des Testelements umfassen, so dass beispielsweise die Überwachung der Testfeldfläche durch das Trägerelement hindurch, welches vollständig oder teilweise als optisch transparentes Trägerelement ausgestaltet sein kann, erfolgen kann. So kann beispielsweise das Sichtfenster von einer Außenseite des Gehäuses her einen Blick auf eine Rückseite eines Trägerelements des Testelements ermöglichen, beispielsweise eine Folienrückseite des Trägerelements, wobei die Testfeldfläche auf einer der Rückseite gegenüberliegenden Vorderseite angeordnet ist, so dass eine Beobachtung durch das Trägerelement hindurch erfolgen kann. Die Testfeldfläche kann dabei beispielsweise, wie oben beschrieben, im Inneren einer Kammer angeordnet sein, so dass durch das Sichtfenster hindurch die Testfeldfläche im Inneren der Kammer beobachtbar ist.

Wie oben beschrieben, kann die Testchemie insbesondere in Form einer oder mehrerer Schichten auf das Trägerelement aufgebracht sein. Zusätzlich zu der mindestens einen Testchemie können dabei weitere Elemente auf dem Trägerelement angeordnet sein. Beispielsweise kann ein mehrschichtiger Aufbau vorgesehen sein, mit mindestens einer Schicht der Testchemie und mindestens einer weiteren Schicht, beispielsweise einer Schicht mit mindestens einem optischen Pigment und/oder mindestens einer Abtrennschicht, mittels derer Bestandteile der Probe abgetrennt werden können, bevor die Probe die Testchemie erreicht. So kann beispielsweise ein Schichtaufbau gewählt werden, bei welchem zunächst auf dem Trägerelement die mindestens eine Testchemie aufgebracht wird, gefolgt von mindestens einer Abtrennschicht und/oder mindestens einer Pigmentschicht, so dass beim Aufbringen der Probe die Probe zunächst die Abtrennschicht und/oder Pigmentschicht durchdringen muss, bevor diese die Testchemie erreicht. Zwischen diesen Möglichkeiten wird begrifflich im Folgenden nicht unterschieden, so dass die Testchemie auch einen mehrschichtigen Aufbau umfassen kann, wobei mindestens eine Schicht die eigentliche Testchemie umfasst und mindestens eine optionale weitere Schicht Testchemie-frei ausgestaltet ist. Insofern ist die Testfeldfläche als Probenaufgabefläche für die Aufgabe der Probe zu verstehen, wobei bei Aufgabe der Probe auf diese Probenaufgabefläche die Probe oder Bestandteile der Probe direkt oder nach Durchdringung einer oder mehrerer Testchemie-freier Schichten zu der Testchemie gelangen können, optional durch eine oder mehrere Abtrenn- und/oder Pigmentschichten. Die Testfeldfläche kann also eine freie Oberfläche der Testchemie umfassen, welche direkt für eine Aufgabe der Probe zugänglich ist, oder kann, alternativ oder zusätzlich, mindestens eine Probenaufgabefläche mindestens einer weiteren, die Testchemie bedeckenden Schicht sein, wobei die mindestens eine weitere Schicht (beispielsweise die mindestens eine Abtrenn- und/oder Pigmentschicht) von der Probe oder zumindest einem Teil der Probe durchdrungen werden kann, um zu der Testchemie zu gelangen. Die optionale mindestens eine Abtrenn- und/oder Pigmentschicht kann beispielsweise dazu dienen, rote Blutkörperchen aus einer Blutprobe abzutrennen und optisch gegenüber dem Sichtfenster abzuschirmen, da diese beispielsweise eine optische Überwachung eines Farbumschlags der Testchemie verhindern und/oder erschweren könnten. So kann die Abtrenn- und/oder Pigmentschicht beispielsweise Pigmente umfassen, welche eine Reflexion von Anregungslicht, welches durch das Sichtfenster und durch das Trägerelement in die Testchemie gerät, ermöglichen. Die Pigmente können beispielsweise Titandioxidpartikel sein oder umfassen.

Das Verfahren kann weiterhin insbesondere derart durchgeführt werden, dass das Gehäuse mindestens eine Kammer bildet. Beispielsweise kann pro analytischem Hilfsmittel genau eine Kammer vorgesehen sein, welche, wie oben ausgeführt, auch eine oder mehrere Öffnungen aufweisen kann. Die Testfeldfläche kann insbesondere einem Innenraum der Kammer zuweisen.

Weist das Gehäuse mindestens eine Kammer auf, so kann das Verfahren insbesondere weiterhin mindestens ein Verfahrensschritt umfassen, in welchem mindestens ein Lanzettenelement zur Generierung der Probe in die Kammer eingebracht wird, insbesondere mindestens ein Microsampler. Sind mehrere Kammern vorgesehen, beispielsweise identische Kammern, beispielsweise eine Kammer pro analytischem Hilfsmittel, so kann beispielsweise jeweils genau ein Lanzettenelement pro Kammer eingebracht werden. Unter einem Lanzettenelement ist allgemein ein Element zu verstehen, welches eingerichtet ist, um einen Einstich und/oder ein Einschnitt in einer Hautoberfläche eines Benutzers zu erzeugen. So kann das Lanzettenelement beispielsweise eine Spitze und/oder eine Schneide und/oder eine scharfe Kannte aufweisen, mittels deren der Einstich und/oder Einschnitt generiert werden kann. Unter einem Microsampler ist ein Lanzettenelement zu verstehen, welches weiterhin mindestens ein Kapillarelement umfasst, beispielsweise mindestens einen Kapillarspalt in einer Oberfläche des Lanzettenelements. Beispielsweise kann sich ein Kapillarspalt von einer Lanzettenspitze des Lanzettenelements aus in einen Lanzettenkörper hinein erstrecken. Das Kapillarelement dient zur Aufnahme und/oder zur Weiterleitung der Probe oder von Bestandteilen derselben.

Die Testchemie kann insbesondere derart stabil ausgewählt werden, dass diese zumindest kurzzeitig stabil gegenüber Temperaturen von 100° C ist, insbesondere gegenüber Temperaturen von 110° C und besonders bevorzugt gegenüber Temperaturen von 120° C. So kann allgemein bezüglich möglicher Testchemien beispielsweise auf den oben beschriebenen Stand der Technik verwiesen werden, beispielsweise auf die oben zitierten WO 2010/094426 A1, WO 2010/094427 A1 oder auf J. Hönes et al.: Diabetes Technology and Therapeutics, Vol. 10, Supplement 1, 2008, S-10 bis S-26. Besonders bevorzugt ist jedoch, wie oben ausgeführt, eine temperaturstabile Testchemie, also eine Testchemie, welche zumindest kurzzeitig stabil gegenüber Temperaturen von 100° C ist, insbesondere gegen Temperaturen von 110° C und besonders bevorzugt gegenüber Temperaturen von 120° C. Unter einer zumindest kurzzeitig stabil gegenüber den genannten Temperaturen ausgestalteten Testchemie wird eine Testchemie verstanden, die bei einer Beaufschlagungsdauer von mindestens 1 Minute, vorzugsweise von mindestens 5 Minuten, mit den genannten Temperaturen in ihrer Aktivität um vorzugsweise weniger als 50 %, insbesondere um weniger als 30 % und besonders bevorzugt um weniger als 20 % abnehmen. Beispielsweise kann die Testchemie, vorzugsweise als Trockenchemie auf dem Trägerelement, zum Test dieser Eigenschaften für die genannten Zeitdauern, beispielsweise für 1 Minute oder 5 Minuten, den genannten Temperaturen ausgesetzt werden. Vor oder nach dieser Temperaturbeaufschlagung wird die Aktivität erfasst. Die Aktivität kann dabei grundsätzlich mittels eines beliebigen, aus dem Stand der Technik bekannten Verfahrens bestimmt werden, da im Rahmen der vorliegenden Definition lediglich die prozentuale Abnahme der Aktivität während der Temperaturbelastung von Relevanz ist. Die Aktivität kann sich dabei insbesondere auf eine Enzymaktivität der Testchemie, insbesondere einer Trockenchemie, beziehen, insbesondere in einem Teststreifen. Beispielsweise sind Verfahren bekannt, die zur Messung der Enzymaktivität das Enzym aus der Testchemie bzw. dem Testelement extrahieren und anschließend beispielsweise mittels einer Ultraviolett-Absorption die Aktivität bestimmen. Diesbezüglich kann beispielsweise auf H. U. Bergmeyer: Methoden der enzymatischen Analyse, Verlag Chemie, 2. Auflage 1970, S. 417 oder auf Banauch et al.: A glucose dehydrogenase for the determination of glucose concentrations in body fluids, Z. Klin. Chem. Klin. Biochem. 1975 Mar; 13(3):101-7 verwiesen werden. Beispielsweise kann für den Test der Stabilität und/oder der Aktivitätsabnahme ein Testelement, beispielsweise ein Teststreifen, mit der Testchemie hergestellt werden. Anschließend kann mit einem üblichen Verfahren die Enzymaktivität eines Enzyms der Testchemie gemessen werden, dann die oben beschriebene Lagerung bei erhöhter Temperatur durchgeführt werden, und anschließend erneut dasselbe Verfahren zur Messung der Enzymaktivität durchgeführt werden. Die Prozedur wird üblicherweise mit einem repräsentativen Kollektiv an Testelementen bzw. Testchemien durchgeführt.

Als Beispiel für temperaturstabile Testchemien kann beispielsweise auf die oben bereits zitierte WO 2007/012494 A1 sowie auf die oben bereits zitierten WO 2010/094426 A1 und WO 2010/094427 A1 verwiesen werden. Die dort dargestellten Testchemien sind auch im Rahmen der vorliegenden Erfindung einsetzbar, allein oder auch in Kombination mit einer oder mehreren anderen Testchemien.

So kann die Testchemie beispielsweise ein Enzym und ein stabiles Coenzym enthalten, welche gemeinsam gelagert sind. Überraschenderweise wurde gefunden, dass mit Hilfe eines stabilen Coenzyms eine Temperaturstabilisierung und/oder eine Langzeitstabilisierung von mehreren Wochen bzw. Monaten bei hoher relativer Feuchte oder sogar in flüssiger Phase und bei erhöhten Temperaturen möglich ist. Diese Erkenntnis ist überraschend, da bekannt ist, dass Enzyme in Gegenwart von nativem Coenzym zwar eine erhöhte Kurzzeitstabilität für einige Stunden besitzen, aber eine geringere Haltbarkeit über einen längeren Zeitraum aufweisen. Gegenüber diesen Erkenntnissen gegenüber dem Stand der Technik war es überraschend, dass ein Enzym in Gegenwart eines stabilen Coenzyms eine deutlich erhöhte Temperatur- und Langzeitstabilität als ein Enzym in Gegenwart eines nativen Coenzyms besitzt, insbesondere da die stabilen Coenzyme eine niedrigere Bindungskonstante mit dem Enzym als das native Coenzym besitzen.

Das durch das offenbarte Verfahren stabilisierte Enzym kann insbesondere ein Coenzymabhängiges Enzym sein. Geeignete Enzyme sind z.B. Dehydrogenasen, ausgewählt aus einer Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder Aminosäure-Dehydrogenase, z. B. L-Aminosäure-Dehydrogenase (E.C.1.4.1.5). Weitere geeignete Enzyme sind Oxidasen, wie etwa Glucoseoxidase (E.C.1.1.3.4) oder Cholesterinoxidase (E.C.1.1.3.6) bzw. Aminotransferasen, wie z.B. Aspartat oder Alanin Aminotransferase, 5'-Nukleotidase oder Creatin-Kinase. Vorzugsweise ist das Enzym Glucose-Dehydrogenase.

Als besonders bevorzugt hat sich der Einsatz einer mutierten Glucose-Dehydrogenase erwiesen. Der Begriff "Mutante", wie er im Rahmen der vorliegenden Anmeldung verwendet wird, bezeichnet eine genetisch veränderte Variante eines nativen Enzyms, welche bei gleicher Anzahl an Aminosäuren eine gegenüber dem Wildtyp-Enzym veränderte Aminosäuresequenz besitzt, d.h. sich in mindestens einer Aminosäure vom Wildtyp-Enzym unterscheidet. Die Einführung der Mutation(en) kann ortsspezifisch oder nicht-ortsspezifisch, bevorzugt ortsspezifisch unter Verwendung von im Fachbereich bekannten rekombinanten Methoden, erfolgen, wobei entsprechend den jeweiligen Anforderungen und Bedingungen mindestens ein Aminosäureaustausch innerhalb der Aminosäuresequenz des nativen Enzyms resultiert. Besonders bevorzugt weist die Mutante eine gegenüber dem Wildtyp-Enzym erhöhte thermische oder hydrolytische Stabilität auf.

Die mutierte Glucose-Dehydrogenase kann die gegenüber der korrespondierenden Wildtyp-Glucose-Dehydrogenase veränderte(n) Aminosäure(n) grundsätzlich an einer beliebigen Position ihrer Aminosäuresequenz enthalten. Vorzugsweise umfasst die mutierte Glucose-Dehydrogenase eine Mutation an mindestens einer der Positionen 96, 170 und 252 der Aminosäuresequenz der Wildtyp-Glucose-Dehydrogenase, wobei Mutanten mit Mutationen an Position 96 und Position 170 bzw. Mutationen an Position 170 und Position 252 besonders bevorzugt sind. Als vorteilhaft hat es sich erwiesen, wenn die mutierte Glucose-Dehydrogenase neben diesen Mutationen keine weiteren Mutationen enthält.

Die Mutation an den Positionen 96, 170 und 252 kann grundsätzlich einen beliebigen Aminosäureaustausch umfassen, welcher zu einer Stabilisierung, z.B. einer Erhöhung der thermischen oder hydrolytischen Stabilität, des Wildtyp-Enzyms führt. Vorzugsweise umfasst die Mutation an Position 96 einen Aminosäureaustausch von Glutaminsäure gegen Glycin, während in Bezug auf Position 170 ein Aminosäureaustausch von Glutaminsäure gegen Arginin oder Lysin, insbesondere ein Aminosäureaustausch von Glutaminsäure gegen Lysin, bevorzugt ist. Was die Mutation an Position 252 anbetrifft, so umfasst diese bevorzugt einen Aminosäureaustausch von Lysin gegen Leucin.

Die mutierte Glucose-Dehydrogenase kann durch Mutation einer aus einer beliebigen biologischen Quelle stammenden Wildtyp-Glucose-Dehydrogenase erhalten werden, wobei der Begriff "biologische Quelle" im Sinne dieser Erfindung sowohl Prokaryoten, wie beispielsweise Bakterien, als auch Eukaryoten, wie beispielsweise Säuger und andere Tiere, umfasst. Vorzugsweise entstammt die Wildtyp-Glucose-Dehydrogenase einem Bakterium, wobei es sich besonders bevorzugt um eine Glucose-Dehydrogenase aus Bacillus megaterium, Bacillus subtilis oder Bacillus thuringiensis, insbesondere aus Bacillus subtilis, handelt.

In einer besonders bevorzugten Ausführungsform der vorliegenden Erfindung handelt es sich bei der mutierten Glucose-Dehydrogenase um eine durch Mutation von Wildtyp-Glucose-Dehydrogenase aus Bacillus subtilis erhaltene Glucose-Dehydrogenase, welche die in SEQ ID NO: 1 (GlucDH_E96G_E170K) oder die in SEQ ID NO: 2 (GlucDH_E170K K252L) dargestellte Aminosäuresequenz besitzt.

Das stabile Coenzym ist vorzugsweise ein gegenüber dem nativen Coenzym chemisch verändertes Coenzym, welches eine im Vergleich zum nativen Coenyzm höhere Stabilität, (z.B. hydrolytische Stabilität) aufweist. Vorzugsweise ist das stabile Coenyzm unter Testbedingungen gegenüber Hydrolyse stabil. Im Vergleich zum nativen Coenzym kann das stabile Coenzym eine verringerte Bindungskonstante für das Enzym aufweisen, beispielsweise eine um den Faktor von 2 oder mehr verringerte Bindungskonstante.

Bevorzugte Beispiele für stabile Coenzyme sind stabile Derivate von Nicotinamid-Adenin-Dinukleotid (NAD/NADH) oder Nicotinamid-Adenin-Dinukleotidphosphat (NADP/NADPH), oder verkürzte NAD-Derivate, z.B. ohne AMP-Teil bzw. mit nicht nukleosidischen Resten, z.B. hydrophoben Resten. Ebenfalls bevorzugt ist als stabiles Coenzym im Sinne der vorliegenden Erfindung die Verbindung der Formel (I).

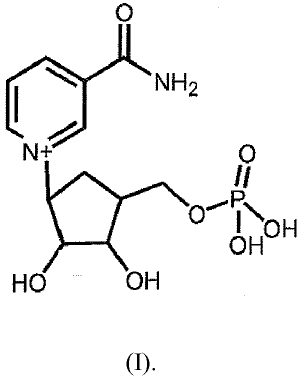

Bevorzugte stabile Derivate von NAD/NADH bzw. NADP/NADPH sind in den zuvor genannten Referenzen beschrieben, auf deren Offenbarung hiermit ausdrücklich Bezug genommen wird. Besonders bevorzugte stabilisierte Coenzyme sind in WO 2007/012494 bzw. US 11/460,366 beschrieben, auf deren Offenbarung hiermit ausdrücklich Bezug genommen wird. Das stabile Coenzym wird besonders bevorzugt aus Verbindungen mit der allgemeinen Formel (II) ausgewählt: 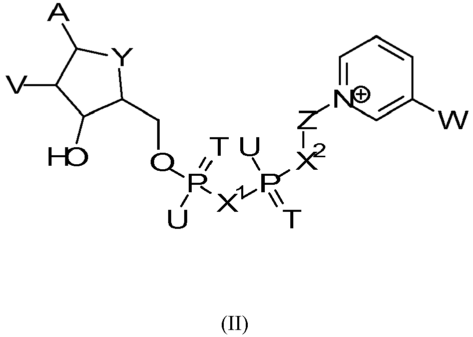 mit
A = Adenin oder ein Analog davon,
T = jeweils unabhängig O, S,
U = jeweils unabhängig OH, SH, BH₃⁻, BCNH₂⁻,
V = jeweils unabhängig OH oder eine Phosphatgruppe, oder zwei Gruppen, die eine cyclische Phosphatgruppe bilden;
W = COOR, CON(R)₂, COR, CSN(R)₂ mit R = jeweils unabhängig H oder C₁-C₂-Alkyl,
X¹, X² = jeweils unabhängig O, CH₂, CHCH₃, C(CH₃)₂, NH, NCH₃,
Y = NH, S, O, CH₂,
Z = ein linearer oder cyclischer organischer Rest ist,
   mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (II) ist Z vorzugsweise ein linearer Rest mit 4-6 C-Atomen, vorzugsweise 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättigter oder ungesättigter carbocyclischer oder heterocyclischer Fünfring, insbesondere eine Verbindung der allgemeinen Formel (III) 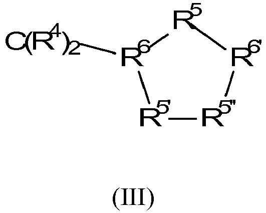
wobei zwischen R⁵' und R⁵" eine Einfach- oder Doppelbindung vorliegen kann, mit
R⁴ = jeweils unabhängig H, F, Cl, CH₃,
R⁵ =CR⁴₂,
wobei R⁵' = O, S, NH, NC₁-C₂-Alkyl, CR⁴₂, CHOH, CHOCH₃, und
R⁵" = CR⁴₂, CHOH, CHOCH₃, wenn zwischen R⁵' und R⁵" eine Einfachbindung vorliegt, und
wobei R⁵'=R⁵"=CR⁴, wenn zwischen R⁵' und R⁵" eine Doppelbindung vorliegt, und
R⁶, R⁶'= jeweils unabhängig CH oder CCH₃.

In einer bevorzugten Ausführungsform enthalten die Verbindungen Adenin oder Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁-C₆-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxy-deoxyribose, 2'-Fluoro-deoxyribose, Hexitol, Altritol bzw. polycyclische Analoga, wie Bicyclo-, LNA- und Tricyclo-Zucker enthalten.

Insbesondere können in den Verbindungen der Formel (II) auch (Di-)-Phosphatsauerstoffe ersetzt sein, beispielsweise isotronisch und/oder isovalent und/oder isoelektronisch ersetzt sein, wie z.B. O⁻ durch S⁻ bzw. BH₃⁻, O durch NH, NCH₃ bzw. CH₂ und =O durch =S.

In den Verbindungen der Formel (II) ist W vorzugsweise CONH₂ oder COCH₃.

In den Gruppen der Formel (III) ist R⁵ vorzugsweise CH₂. Weiterhin ist bevorzugt, dass R⁵' ausgewählt ist aus CH₂, CHOH und NH. In einer besonders bevorzugten Ausführungsform sind R⁵' und R⁵" jeweils CHOH. In noch einer weiteren bevorzugten Ausführungsform ist R⁵' NH und R⁵" CH₂.

In der am stärksten bevorzugten Ausführungsform handelt es sich bei dem stabilen Coenzym um carbaNAD, wie beispielsweise unter anderem in der vorstehend bereits genannten WO 2007/012494 beschrieben..

Die bevorzugte Testchemie ist insbesondere derart ausgestaltet, dass die in dieser enthaltene Enzyme Langzeit-stabilisiert sind. Dies bedeutet, dass man das mit einem stabilen Coenzym stabilisierte Enzym, z.B. als Trockensubstanz, beispielsweise über eine Dauer von mindestens zwei Wochen, vorzugsweise von mindestens vier Wochen und besonders bevorzugt von mindestens acht Wochen lagert und wobei die Enzymaktivität vorzugsweise um weniger als 50 %, besonders bevorzugt weniger als 30 % und am meisten bevorzugt um weniger als 20 % bezüglich des Ausgangswerts der Enzymaktivität abnimmt.

Durch die genannte Stabilisierung ist es möglich, das mit einem stabilen Coenzym stabilisierte Enzym auch ohne Trocknungsreagenz für eine lange Zeit, wie oben angegeben, und/oder bei hohen Temperaturen, wie oben angegeben, zu lagern. Weiterhin kann das stabilisierte Enzym auch bei einer hohen relativen Luftfeuchtigkeit, z.B. einer relativen Luftfeuchtigkeit von mindestens 50 % gelagert werden, wobei die Enzymaktivität vorzugsweise um weniger als 50 %, besonders bevorzugt weniger als 30 % und am meisten bevorzugt weniger als 20 % bezüglich des Ausgangswerts abnimmt.

Die Lagerung des mit einem stabilen Coenzym stabilisierten Enzyms kann einerseits als Trockensubstanz und andererseits in Flüssigphase erfolgen. Bevorzugt erfolgt die Lagerung des stabilisierten Enzyms auf oder in einem Testelement, das zur Bestimmung eines Analyten geeignet ist. Das mit einem stabilen Coenzym stabilisierte Enzym ist dabei Bestandteil der bevorzugten Testchemie, welche gegebenenfalls noch weitere Bestandteile wie etwa Salze, Puffer, etc. enthalten kann. Vorzugsweise ist die Testchemie dabei frei von einem Mediator.

Das mit einem stabilen Coenzym stabilisierte Enzym kann allgemein zum Nachweis von Analyten, beispielsweise Parametern in Körperflüssigkeiten wie etwa Blut, Serum, Plasma oder Urin bzw. in Abwasserproben oder Lebensmitteln eingesetzt werden.

Als Analyten können beliebige biologische oder chemische Substanzen bestimmt werden, die durch eine Redoxreaktion nachgewiesen werden können, z.B. Substanzen, bei denen es sich um Substrate eines Coenzym-abhängigen Enzyms handelt oder Coenzym-abhängige Enzyme selbst. Bevorzugte Beispiele für Analyten sind Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc. Bevorzugt ist der Analyt Glucose. Besonders bevorzugt ist hierbei der Nachweis von Glucose mit Hilfe von Glucose-Dehydrogenase (GlucDH).

Die Veränderung des stabilen Coenzyms durch Reaktion mit dem Analyten kann grundsätzlich auf beliebige Art und Weise nachgewiesen werden. Hier können grundsätzlich alle aus dem Stand der Technik bekannten Methoden zum Nachweis enzymatischer Reaktionen eingesetzt werden. Vorzugsweise wird jedoch die Veränderung des Coenzyms durch optische Methoden nachgewiesen. Optische Nachweismethoden umfassen beispielsweise die Messung von Absorption, Fluoreszenz, Circulardichroismus (CD), optische Rotationsdispersion (ORD), Refraktometrie etc.

Ein optisches Nachweisverfahren, welches im Rahmen der vorliegenden Anmeldung bevorzugt Anwendung findet, ist die Photometrie. Zur photometrischen Messung einer Veränderung des Coenzyms infolge Umsetzung mit dem Analyten bedarf es indessen der zusätzlichen Anwesenheit mindestens eines Mediators, welcher die Reaktivität des reduzierten Coenzyms erhöht und eine Übertragung von Elektronen auf einen geeigneten optischen Indikator oder ein optisches Indikatorsystem ermöglicht.

Als Mediatoren, welche für die Zwecke der vorliegenden Erfindung geeignet sind, kommen u.a. Nitrosoaniline, wie beispielsweise [(4-Nitrosophenyl)imino]dimethanol-Hydrochlorid, Chinone, wie beispielsweise Phenanthrenchinone, Phenanthrolinchinone oder Benzo[h]-chinolinchinone, Phenazine, wie beispielsweise 1-(3-Carboxypropoxy)-5-ethylphenazinium-trifluormethansulfonat, oder/und Diaphorase (EC 1.6.99.2) in Frage. Bevorzugte Beispiele für Phenanthrolinchinone umfassen 1,10-Phenanthrolin-5,6-chinone, 1,7-Phenanthrolin-5,6-chinone, 4,7-Phenanthrolin-5,6-chinone sowie deren N-alkylierte oder N,N'-dialkylierte Salze, wobei im Falle N-alkylierter bzw. N,N'-dialkylierter Salze Halogenide, Trifluormethansulfonat oder andere die Löslichkeit erhöhende Anionen als Gegenion bevorzugt sind.

Als optischer Indikator oder als optisches Indikatorsystem kann eine beliebige Substanz verwendet werden, welche reduzierbar ist und bei Reduktion eine detektierbare Änderung ihrer optischen Eigenschaften, wie beispielsweise Farbe, Fluoreszenz, Remission, Transmission, Polarisation oder/und Brechungsindex, erfährt. Die Bestimmung des Vorhandenseins oder/und der Menge des Analyten in der Probe kann mit dem bloßen Auge oder/und mittels einer Detektionsvorrichtung unter Verwendung eines dem Fachmann geeignet erscheinenden photometrischen Verfahrens erfolgen. Vorzugsweise werden Heteropolysäuren, und insbesondere 2,18-Phosphormolybdänsäure, als optische Indikatoren verwendet, die zum entsprechenden Heteropolyblau reduziert werden.

Besonders bevorzugt wird die Veränderung des Coenzyms durch Messung der Fluoreszenz nachgewiesen. Die Fluoreszenzmessung ist hochsensitiv und ermöglicht den Nachweis selbst geringer Konzentrationen des Analyten in miniaturisierten Systemen.

Alternativ kann die Veränderung des Coenzyms auch elektrochemisch unter Verwendung eines geeigneten Testelements, wie beispielsweise eines elektrochemischen Teststreifens, nachgewiesen werden. Voraussetzung hierfür ist wiederum die Verwendung geeigneter Mediatoren, welche vom reduzierten Coenzym durch Übertragung von Elektronen in eine reduzierte Form überführt werden können. Die Bestimmung des Analyten erfolgt über eine Messung des zur Reoxidation des reduzierten Mediators benötigten Stromes, welcher mit der Konzentration des Analyten in der Probe korreliert. Beispiele für Mediatoren, welche für elektrochemische Messungen verwendet werden können, umfassen insbesondere die vorstehend erwähnten, für photometrische Messungen eingesetzten Mediatoren.

Ein besonders bevorzugtes Testformat, umfasst die Verwendung des Enzyms Glucose-Dehydrogenase mit einem stabilen NAD-Derivat zum Nachweis von Glucose, wobei ein Derivat des reduzierten Coenzyms NADH gebildet wird. Der Nachweis von NADH erfolgt durch optische Methoden, z.B. durch photometrische oder fluorometrische Bestimmung nach UV-Anregung. Ein besonders bevorzugtes Testsystem ist in US 2005/0214891 beschrieben, auf das hier ausdrücklich Bezug genommen wird.

Insbesondere kann die stabile Testchemie derart ausgestaltet sein, dass diese ein mit einem stabilen Coenzym stabilisiertes Enzym umfasst, wobei das stabilisierte Enzym bei einer Lagerung von vorzugsweise mindestens zwei Wochen, besonders bevorzugt mindestens vier Wochen und am meisten bevorzugt mindestens acht Wochen bei einer Temperatur von vorzugsweise mindestens 20° C, besonders bevorzugt mindestens 25° C und am meisten bevorzugt mindestens 30° C, gegebenenfalls bei hoher Luftfeuchtigkeit und ohne Trockenreagenz eine Abnahme der enzymatischen Aktivität von weniger als 50 %, vorzugsweise weniger als 30 % und am meisten bevorzugt weniger als 20 % gegenüber dem Ausgangswert aufweist.

Wie oben ausgeführt, kann das Testelement insbesondere mindestens ein Trägerelement aufweisen, wobei die Testchemie vorzugsweise mit dem Trägerelement verbunden ist. Dieses Verbinden kann beispielsweise dadurch erfolgen, dass die Testchemie direkt oder indirekt in Form mindestens einer Testchemieschicht auf das Trägerelement aufgebracht ist, was beispielsweise auch im Rahmen des oben beschriebenen Verfahrensschritts a) erfolgen kann. Beispielsweise kann das Aufbringen durch ein Verfahren erfolgen, ausgewählt aus der Gruppe bestehend aus Rakeln, Drucken (insbesondere Siebdruck, Schablonendruck, Tampondruck) und Aufschleudern.

Das Trägerelement kann, wie oben beschrieben, insbesondere ganz oder teilweise aus mindestens einem Kunststoffmaterial hergestellt sein. Insbesondere kann es sich bei diesem Kunststoffmaterial um ein Kunststoffmaterial mit einer Erweichungstemperatur, bestimmbar nach DIN EN ISO 306, von mindestens 100° C, vorzugsweise von mindestens 110° C und besonders bevorzugt von mindestens 120° C handeln, beispielsweise mit einer Erweichungstemperatur von mindestens 130° C oder sogar mindestens 140° C oder mindestens 150° C. Beispiele derartiger Kunststoffe sind Acrylnitril-Butadien-Styrol (ABS), Polymethylmethacrylat (PMMA), Polypropylen (PP), Polyester und Polycarbonat (PC) oder Kombinationen der genannten und/oder anderer Kunststoffe. Auch andere Kunststoffe sind jedoch grundsätzlich einsetzbar. Insbesondere kann das Gehäuseteil, mit welchem das Testelement während des Formgebungsverfahrens verbunden wird, vollständig oder teilweise aus einem derartigen Kunststoffmaterial als Gehäusewerkstoff hergestellt sein.

Das Trägerelement kann insbesondere mindestens ein Folienelement umfassen, wobei bezüglich des Folienelements auf die obige Definition verwiesen werden kann. Insbesondere kann es sich hierbei um eine Kunststofffolie handeln. Diese Folie kann einschichtig oder auch mehrschichtig aufgebaut sein. Das Trägerelement kann eine oder mehrere Testchemien tragen. Weiterhin kann das Trägerelement, alternativ oder zusätzlich, die mindestens eine Testchemie für mehrere analytische Hilfsmittel tragen. So kann beispielsweise ein Trägerelement für mehrere analytische Hilfsmittel vorgesehen sein, so dass mehrere analytische Hilfsmittel sich ein Trägerelement oder Teile desselben teilen. Beispielsweise kann das Trägerelement eine durchgängige Testchemie tragen, wobei verschiedene Oberflächenbereiche dieser Testchemie Testfeldflächen für verschiedene analytische Hilfsmittel bilden. Beispielsweise kann eine Trägerfolie vorgesehen sein, welche großflächig mit Testchemie beschichtet ist, wobei mindestens ein erster Bereich einer Oberfläche der Testchemie für ein erstes analytisches Hilfsmittel vorgesehen ist, und mindestens ein zweiter Bereich der Oberfläche als Testfeldfläche für mindestens ein zweites analytisches Hilfsmittel. Alternativ zu einer durchgängigen Testchemieschicht, welche für mehrere analytische Hilfsmittel Testfeldflächen bereitstellt, können auch unterschiedliche Testchemieschichten vorgesehen sein, beispielsweise indem auf einem Trägerelement mehrere nebeneinanderliegende Testchemieschichten aufgebracht sind, beispielsweise durch ein Druckverfahren, ein Rakelverfahren, ein Dispenserverfahren, ein Aufschleudern oder ein anderes Beschichtungsverfahren, wobei die mehreren Testchemieschichten jeweils Testfeldflächen für ein oder mehrere analytische Hilfsmittel bereitstellen können. So kann beispielsweise eine gemeinsame Trägerfolie vorgesehen sein, auf welcher nebeneinander mindestens eine erste Testchemieschicht und mindestens eine zweite Testchemieschicht aufgedruckt sind, wobei die erste Testchemieschicht mit ihrer Oberfläche mindestens eine erste Testfeldfläche für mindestens ein erstes analytisches Hilfsmittel bereitstellt und wobei die zweite Testchemieschicht mit ihrer Oberfläche mindestens eine zweite Testfeldfläche für mindestens ein zweites analytisches Hilfsmittel bereitstellt.

Das Trägerelement kann insbesondere als scheibenförmiges Trägerelement ausgestaltet werden. Beispielsweise kann das Trägerelement in Form einer rechteckigen, polygonalen oder runden Scheibe ausgestaltet werden. Besonders bevorzugt ist, wie unten näher beschrieben wird, die Verwendung mindestens eines streifenförmigen Trägerelements und/oder mindestens eines ringförmigen Trägerelements (beispielsweise mindestens eines kreisringförmigen Trägerelements) und/oder mindestens eines kreisscheibenförmigen Trägerelements. Beispielsweise können kreisringförmige Trägerelemente hergestellt werden. Die Testchemie kann als Beschichtung auf das Trägerelement aufgebracht werden, wobei die Beschichtung Testfeldflächen für die analytischen Hilfsmittel bereitstellt. Die analytischen Hilfsmittel können beispielsweise konzentrisch um einen Mittelpunkt der Scheibe, insbesondere der Kreisscheibe und besonders bevorzugt, der Kreisringscheibe angeordnet sein.

Das Gehäuseteil kann insbesondere zumindest teilweise aus mindestens einem Gehäusewerkstoff hergestellt werden, welches vorzugsweise ausgewählt ist aus der Gruppe bestehend aus: einem Polycarbonat; einem Polyester; einem Acrylnitril-Butadien-Styrol, einem Cyclo-Olefin-Copolymer; einem Polymethylmethacrylat; einem Polystyrol; einem Polyethylenterephthalat.

Das beschriebene Verfahren kann derart durchgeführt werden, dass genau ein analytisches Hilfsmittel hergestellt wird. Erfindungsgemäß wird das Verfahren jedoch derart durchgeführt, dass mehrere analytische Hilfsmittel hergestellt werden, wobei die analytischen Hilfsmittel in einem analytischen Magazin enthalten sind. Unter dem Ausdruck "sind enthalten" ist dabei allgemein die Möglichkeit zu verstehen, dass die analytischen Hilfsmittel beispielsweise in einer äußeren Umhüllung des Magazins aufgenommen sind, beispielsweise in einem Innenraum des Magazins. Gemäß der vorliegenden Beschreibung können die analytischen Hilfsmittel jedoch derart in dem Magazin enthalten sein, dass diese feste Bestandteile des Magazins sind, beispielsweise indem die analytischen Hilfsmittel fest, insbesondere starr, miteinander verbunden sind und indem beispielsweise die Gehäuseteile der analytischen Hilfsmittel Bestandteile eines Magazingehäuses des Magazins sind.

Die analytischen Hilfsmittel eines Magazins werden erfindungsgemäß gleichzeitig hergestellt. Dies erfolgt dadurch, dass bei dem oben beschriebenen Formgebungsverfahren, beispielsweise bei dem Kunststoff-Formgebungsverfahren, gleichzeitig die Gehäuseteile der analytischen Hilfsmittel hergestellt und auch gleichzeitig miteinander verbunden werden. Dies kann beispielsweise dadurch erfolgen, dass die Gehäuseteile in ein und demselben Formnest und/oder in verschiedenen Formnestern ein und desselben Werkzeugs hergestellt werden. Sind mehrere Formnester für die Gehäuseteile vorgesehen, so sind diese beispielsweise fluidisch miteinander verbunden, so dass diese beispielsweise während eines Formgebungsverfahrens durch den Gehäusewerkstoff miteinander verbunden werden können.

In einem weiteren Aspekt der vorliegenden Beschreibung wird ein analytisches Hilfsmittel zum Nachweis mindestens eines Analyten in einer Probe, insbesondere einer flüssigen Probe und besonders bevorzugt einer Probe einer Körperflüssigkeit, vorgeschlagen. Das analytische Hilfsmittel kann insbesondere nach einem Verfahren gemäß einer oder mehreren der oben beschriebenen oder nachfolgend noch beschriebenen Ausgestaltung herstellbar sein. Dementsprechend kann für mögliche Ausgestaltungen des analytischen Hilfsmittels auf die möglichen Ausgestaltungen des Verfahrens verwiesen werden und umgekehrt. Das analytische Hilfsmittel umfasst mindestens ein Gehäuse mit mindestens einem Gehäuseteil und mindestens einem Testelement. Das Testelement kann beispielsweise gemäß der obigen Beschreibung ausgestaltet sein und kann insbesondere mindestens ein Trägerelement umfassen. Weiterhin umfasst das Testelement mindestens eine Testchemie. Das Testelement ist mittels eines Formgebungsverfahrens des Gehäuseteils mit dem Gehäuseteil verbunden.

Für weitere mögliche Ausgestaltungen kann auf die obige oder nachfolgende Beschreibung verwiesen werden, insbesondere die Beschreibung des Verfahrens. So kann insbesondere das Testelement derart mit dem Gehäuseteil verbunden sein, dass das Testelement an mindestens einer Fläche auf dem Gehäuseteil aufliegt. Das Testelement kann insbesondere zumindest teilweise in das Gehäuseteil eingebettet sein. Das Testelement kann insbesondere mit dem Gehäuseteil in einer Weise verbunden sein, ausgewählt aus der Gruppe bestehend aus einer stoffschlüssigen Verbindung.

Das Testelement kann insbesondere mit den Gehäuseteil in einer Weise verbunden sein, ausgewählt aus der Gruppe bestehend aus: einer stoffschlüssigen Verbindung, einer formschlüssigen Verbindung, und einer kraftschlüssigen Verbindung. Das Gehäuseteil kann insbesondere mindestens einen Gehäusewerkstoff umfassen, wobei der Gehäusewerkstoff vorzugsweise mindestens einen Kunststoffwerkstoff umfasst, beispielsweise einen oder mehrere der oben genannten Kunststoffwerkstoffe. Das Testelement kann insbesondere durch Hinterspritzen und/oder Umspritzen mit dem Gehäuseteil verbunden sein. Das Testelement kann insbesondere derart mit dem Gehäuseteil verbunden sein, dass ein Teil der Testchemie freiliegt, für eine Aufgabe der Probe, wobei dieser Teil der Testchemie beispielsweise mindestens eine Testfeldfläche bilden kann. Für mögliche Ausgestaltungen der Testfeldfläche kann auf die obige Beschreibung verwiesen werden. Insbesondere kann das Testelement derart mit dem Gehäuseteil verbunden sein, dass mindestens eine Oberfläche der Testchemie als Testfeldfläche für eine Aufgabe der Probe von einem Gehäusewerkstoff des Gehäuseteils unbedeckt verbleibt, also nicht mit Gehäusewerkstoff des Gehäuseteils bedeckt ist. Das Gehäuse kann insbesondere derart ausgestaltet sein, dass mindestens ein Sichtfenster in dem Gehäuse gebildet ist, wobei die Testfeldfläche durch das Sichtfenster des Gehäuses optisch überwachbar ist, insbesondere von außerhalb des Gehäuses. Das Gehäuse kann insbesondere mindestens eine Kammer aufweisen, wobei die Testfeldfläche vorzugsweise einem Innenraum der Kammer zuweist. Weiterhin kann das analytische Hilfsmittel mindestens ein Lanzettenelement umfassen, beispielsweise mindestens ein Lanzettenelement, welches in einer Kammer aufgenommen ist, insbesondere mindestens einen Microsampler.

Das Testelement kann also insbesondere unmittelbar, also ohne Zwischenschaltung eines oder mehrerer Verbindungselemente und/oder Verbindungswerkstoffe, mit dem Gehäuseteil verbunden sein. Insbesondere kann das Testelement Klebstoff-frei mit dem Gehäuseteil verbunden sein. Das Testelement kann insbesondere zumindest partiell in das Gehäuseteil eingebettet sein, wobei bezüglich der verschiedenen Möglichkeiten auf die obige Beschreibung verwiesen werden kann.

Das analytische Hilfsmittel kann insbesondere mindestens eine Kammer umfassen, wobei das Testelement mindestens eine der Kammer zuweisende Testfeldfläche aufweist, also eine Oberfläche der Testchemie, wobei die Testfeldfläche von der Kammer aus für eine Aufgabe der Probe zugänglich ist. Die Testfeldfläche kann insbesondere derart ausgestaltet sein, dass diese von einer Wand der Kammer zumindest teilweise umrahmt wird, beispielsweise zumindest teilweise von einer Wand der Kammer überlappt wird, wobei die Wand der Kammer beispielsweise auf der Testfeldfläche aufliegen kann.

Weiterhin kann das Gehäuse, alternativ oder zusätzlich, mindestens ein Sichtfenster aufweisen, wobei die Testfeldfläche durch das Sichtfenster des Gehäuses optisch überwachbar ist. Für mögliche Ausgestaltungen kann auf die obige Beschreibung verwiesen werden.

Wie oben ausgeführt, kann das analytische Hilfsmittel, zusätzlich zu der mindestens einen Testchemie, weiterhin mindestens ein Lanzettenelement zur Generierung der Probe aufweisen, insbesondere mindestens einen Microsampler. Das Lanzettenelement kann beispielsweise beweglich zu dem Gehäuse gelagert sein, beispielsweise in dem Gehäuse, insbesondere in mindestens einer Kammer des Gehäuses. Die bewegliche Lagerung kann insbesondere derart ausgestaltet sein, dass das Lanzettenelement mindestens eine Lanzettenbewegung durchführen kann, also eine Bewegung, bei welcher die Probe generiert wird. Diese Lanzettenbewegung kann beispielsweise in einer Lanzettenrichtung erfolgen, bei welcher mindestens eine Spitze und/oder Schneide des Lanzettenelements die Kammer verlässt, beispielsweise durch eine entsprechende Öffnung. Die Lanzettenbewegung kann beispielsweise von einem Aktor, welcher Bestandteil des analytischen Hilfsmittels oder eines separaten Geräts sein kann, angetrieben werden. Beispielsweise kann zu diesem Zweck mindestens ein Aktor an einem der Lanzettenspitze oder Lanzettenschneide abgewandten Ende an die Lanzette angreifen. Dieser Aktor kann beispielsweise durch eine Aktoröffnung in die Kammer des analytischen Hilfsmittels eintreten. Der Aktor kann beispielsweise durch einen entsprechenden Federmechanismus angetrieben sein. Bezüglich möglicher Ausgestaltungen derartiger Aktoren, die dem Fachmann grundsätzlich bekannt sind, kann auf den Stand der Technik verwiesen werden.

Das analytische Hilfsmittel kann insbesondere derart eingerichtet sein, dass das Lanzettenelement nach Durchführung der Lanzettenbewegung zurück in das Gehäuse bewegbar ist. Zu diesem Zweck kann die Lanzette beispielsweise mindestens ein Kopplungselement umfassen, welches an einen Aktor ankoppelbar ist, wobei mittels des Aktors eine Lanzettenbewegung und eine Remagazinierungsbewegung in einer der Lanzettenbewegung entgegengesetzten Richtung durchführbar ist, wobei bei der Remagazinierung das Lanzettenelement wieder in dem Gehäuse gelagert werden kann. Während oder nach der Zurückbewegung kann die von dem Lanzettenelement aufgenommene Probe auf das Testelement übertragbar sein. Diese Übertragung kann auf verschiedene Weise erfolgen. Beispielsweise kann das Lanzettenelement, beispielsweise der Microsampler, derart dicht an dem Testelement, insbesondere an einer Testfeldfläche des Testelements vorbei geführt werden, dass die Probe auf die Testfeldfläche übertragen wird. Alternativ oder zusätzlich kann auch mindestens ein weiterer Aktor vorgesehen sein, welcher die Übertragung bewirkt, beispielsweise indem das Lanzettenelement auf die Testfeldfläche gepresst wird. Alternativ oder zusätzlich kann wiederum beispielsweise auch das Gehäuse derart geformt sein, dass bei der Zurückbewegung des Lanzettenelements in das Gehäuse, also beispielsweise bei der Remagazinierungsbewegung, die Lanzette kurzfristig an die Testfeldfläche angenähert wird, so dass ein Übertragung stattfindet. Dies kann beispielsweise durch eine entsprechend gekrümmte Führung des Lanzettenelements in dem Gehäuse bewirkt werden.

Wie oben ausgeführt, kann das analytische Hilfsmittel insbesondere derart ausgestaltet sein, dass das Gehäuse mindestens ein von einer Außenseite des Gehäuses zugängliches Sichtfenster aufweist. Mindestens eine Eigenschaftsänderung des Testelements, insbesondere mindestens eine Farbänderung und/oder mindestens eine Änderung mindestens einer optischen Eigenschaft, kann durch das Sichtfenster von außen detektierbar sein. Für mögliche Ausgestaltungen des Sichtfensters kann auf die obige Beschreibung verwiesen werden. Das Sichtfenster kann insbesondere von einem Rahmen des Gehäuses, insbesondere des Gehäuseteils, zumindest teilweise umschlossen sein. Dieser Rahmen kann beispielsweise unmittelbar auf dem Testelement aufliegen. Wie oben beschrieben, kann das Sichtfenster insbesondere derart ausgestaltet sein, dass eine optische Überwachung der Testchemie durch das Sichtfenster und durch mindestens ein Trägerelement des Testelements hindurch ermöglicht ist. So kann das Sichtfenster insbesondere derart ausgestaltet sein, dass mindestens eine Testfeldfläche des Testelements durch mindestens ein optisch zumindest teilweise transparentes Trägerelement des Testelements hindurch beobachtbar ist. Unter einer Beobachtung ist in diesem Fall beispielsweise eine Detektion mindestens einer optischen Eigenschaftsänderung der Testchemie zu verstehen,

In einem weiteren Aspekt der vorliegenden Erfindung wird ein analytisches Magazin gemäß Anspruch 11 vorgeschlagen. Im Folgenden wird ein analytisches Magazin beschrieben, welches eine Mehrzahl analytischer Hilfsmittel gemäß einem oder mehreren der zuvor beschriebenen Ausgestaltungen oder gemäß einer oder mehreren der nachfolgend noch beschriebenen Ausführungsbeispiele umfasst. Die analytischen Hilfsmittel können insbesondere fest miteinander verbunden sein. Insbesondere können die analytischen Hilfsmittel starr miteinander verbunden sein, so dass deren relative Lage festgelegt ist. Insbesondere kann das analytische Magazin ein Magazingehäuse aufweisen, wobei die Gehäuse der analytischen Hilfsmittel, insbesondere die Gehäuseteile, Bestandteile des Magazingehäuses sein können. So können beispielsweise die Gehäuseteile der analytischen Hilfsmittel aneinander grenzende oder auf andere Weise miteinander verbundene Abschnitte des Magazingehäuses sein.

Das analytische Magazin kann insbesondere eine Kreisscheibenform und/oder eine Kreisringform aufweisen, wobei die analytischen Hilfsmittel beispielsweise radial in dem analytischen Magazin angeordnet sind. Alternativ oder zusätzlich zu einer Kreisringform sind jedoch auch andere Formen möglich, beispielsweise eine Scheibenform, eine Stangenform, eine Streifenform oder andere Formen.

Das im Rahmen der vorliegenden Offenbarung vorgeschlagene Verfahren, das analytische Hilfsmittel und das analytische Magazin weisen gegenüber bekannten Verfahren und Vorrichtungen eine Vielzahl von Vorteilen auf Insbesondere können die oben beschriebenen Nachteile bekannter Verfahren und Vorrichtungen zumindest weitgehend vermieden werden. Weiterhin kann das Verfahren auch sehr kostengünstig und rationell durchgeführt werden, da eine Vielzahl von Arbeitsschritten gegenüber herkömmlichen Herstellungsverfahren entfallen können. Insbesondere kann eine Aufbringung eines Klebstoffs auf Gehäuseteile und/oder auf das Testelement entfallen, wenn die Testchemie vorzugsweise als ein zusammenhängendes Stück, beispielsweise in Form eines Testelements mit einem länglichen Streifen für ein Flachmagazin in gerader Anordnung und/oder in einer Trommel mit einer Anordnung für Testfelder am Umfang, oder als ein Ring für ein Magazin in runder Scheibenanordnung, bereitgestellt und mit dem Gehäuseteil verbunden werden. Insbesondere kann das Testelement als Einlegeteil in ein Spritzgußwerkzeug eingelegt und mit dem Gehäusewerkstoff des Gehäuses, vorzugsweise des Magazingehäuses, umspritzt werden. Dabei bleibt vorzugsweise ein Sichtfenster, also allgemein ein Sichtbereich, für die jeweilige Testfeldfläche frei, wobei beispielsweise eine Trägerfolie des Testelements als Messfenster genutzt werden kann. Das Formgebungsverfahren, insbesondere das Kunststoff-Formgebungsverfahren und besonders bevorzugt das Spritzgußverfahren, muss also keine optischen Qualitäten aufweisen. Die Testchemie kann sogar grundsätzlich mit einem Gehäusewerkstoff in Form eines geschwärzten Materials kombiniert werden, beispielsweise umspritzt werden, was insbesondere die Unterdrückung von Streulicht bei einer Messung erleichtern kann. Dementsprechend kann der Gehäusewerkstoff vorzugsweise als optisch intransparentes Material ausgestaltet sein, beispielsweise als geschwärztes Material, vorzugsweise mit einer Transparenz von weniger als 5%, insbesondere von weniger als 1 % und besonders bevorzugt von weniger als 0,5%, beispielsweise im Spektralbereich von 400 nm bis 800 nm.

Das Testelement, beispielsweise mit einem zusammenhängenden Stück einer Testchemie, kann beispielsweise entweder einseitig hinterspritzt werden, so dass vorzugsweise die volle Fläche der eigentlichen Testchemie schichtfrei bleibt, oder, beispielsweise falls die Wandstärken auf beiden Seiten des Trägerelements hinreichend dick sind, um einen Spritzgußvorgang zu ermöglichen (beispielsweise ≥ 0,4 mm) - kann die Testchemieschicht nur in den einzelnen Testfeldflächen offen liegen, und die Zwischenräume zwischen den Testfeldflächen können vollständig mit Kunststoff ausgefüllt und/oder von Kunststoff umgeben sein.

Insbesondere die oben beschriebene bevorzugte Testchemie, welche auch als cNAD-Testchemie bezeichnet wird, ist für das vorgeschlagene Verfahren und die vorgeschlagenen Vorrichtungen besonders geeignet, da diese zumindest kurzzeitig Temperaturen von typischerweise bis zum 120 °C standhält. Die Verarbeitungstemperaturen der für die vorgeschlagenen analytischen Hilfsmittel und analytischen Magazine typischerweise brauchbaren Kunststoffe liegt in der Regel zwar oberhalb von 120 °C. Diejenigen Bereiche, die als Testchemie hinterher verfügbar und funktionstüchtig sein müssen, nämlich die Testfeldflächen, liegen jedoch vorzugsweise bei dem vorgeschlagenen Verfahren an einer deutlich kühleren Werkzeugwand an, beispielsweise einer Werkzeugwand, welche maximal eine Temperaturen von 110 °C erreicht, da diese von Gehäusewerkstoff, beispielsweise von Spritzgußmasse freibleiben sollten.

Da derartige Formteile medizinischer Verbrauchmaterialien typischerweise generell frei von Formtrennmitteln sein sollten, ist in der Regel auch keine Kontamination der Testchemie mit Fremdstoffen zu befürchten.

Das Testelement kann beispielsweise, wie oben ausgeführt, mindestens ein Trägerelement, beispielsweise mindestens eine Trägerfolie, umfassen. Beispielsweise können Trägerfolien aus Polycarbonat und/oder einem Polyester verwendet werden. Auch als Gehäusewerkstoff kann beispielsweise ein Polycarbonat verwendet werden, oder es kann grundsätzlich für das mindestens eine Gehäuseteil ein zu dem Trägerelement identischer oder zumindest chemisch ähnlicher Werkstoff verwendet werden. Auf diese Weise kann eine gute Verbindung zwischen dem Trägerelement, beispielsweise als Einlegeteil, und dem Gehäuseteil, beispielsweise als Spritzgußteil, gewährleistet werden.

Ein weiterer vorteilhafter Aspekt besteht darin, dass bei miniaturisierten Messsystemen die aufsummierten und typischerweise unvermeidlichen Maßabweichungen in der Regel nur sehr klein sein dürfen, da sonst beispielsweise eine Messoptik, die auf kurzem Arbeitsabstand arbeiten muss, nicht in jedem Fall das Objekt (beispielsweise die Testfeldfläche und/oder die Testchemie) im Fokus hat. So liegen typische Schärfentiefen optischer Nachweissysteme bei maximal ± 0,15 mm. Bei einer montierten Testchemie kommen in der Regel zu den unvermeidbaren formungebundenen Maßen noch die Dickenmaße des typischerweise verwendeten Klebers und die Dicke der Trägerfolie hinzu, deren ebenfalls noch unvermeidbare Abweichungen über die Serie leicht die noch verfügbare Schärfentiefe einer Messoptik übersteigen können. Immerhin muss bei herkömmlichen Geräten in dem Gerät selbst, einschließlich der Messoptik, die selbst typischerweise nicht frei von Messabweichungen bleibt, auch noch ein Montagespielraum verbleiben. Diese Nachteile herkömmlicher miniaturisierter Messsysteme lassen sich durch das vorgeschlagene analytische Hilfsmittel und das erfindungsgemäße analytische Magazin vermeiden oder zumindest deutlich reduzieren. Beispielsweise im Fall einer direkten Umspritzung oder Hinterspritzung des Testelements bleibt in der Regel nur das formungebundene Maß der Magazindicke übrig, die typischerweise in noch verträglichen Grenzen, beispielsweise mit einer Toleranz mit ± 0,05 mm, konstant gehalten werden kann, so dass für das Gerät noch typischerweise ± 0,1 mm an Toleranz für Maßabweichungen verbleiben. Im Zusammenhang mit dem Begriff der "formungebundenen Maße" ist darauf hinzuweisen, dass es sich hier in der Regel um eine Maßkette handelt, welche am Magazin von außen angreift, hin zur Testchemie, welche im Inneren des Magazins angeordnet ist. Folglich beinhaltet diese Maßkette in der Regel Dimensionen, welche erst im Zusammenwirken mehrerer Formteile des Magazins, beispielsweise zweier Formhälften des Magazins, entstehen. In diesem Zusammenhang lassen sich beispielsweise alle Strukturen, welche aus den Werkzeugstrukturen des Formgebungsprozesses resultieren, welche direkt in die Werkzeugwand eingearbeitet sind, als formgebunden bezeichnen. Alle Strukturen hingegen, welche aus dem zusammenwirken verschiedener, gegeneinander beweglicher Werkzeugwände entstehen, lassen sich als formungebunden bezeichnen. Derartige formungebundene Strukturen tragen in der Regel nicht nur die Abweichungen der Werkzeugherstellung, die Varianzen der Formmasse und die Einflüsse des Formgebungsprozesses in sich, sondern obendrein in der Regel noch ein Spiel, welches für die Beweglichkeit der Werkzeugteile gegeneinander erforderlich ist sowie in der Regel die Einflüsse einer Formgebungsmaschine, welche die Bestandteile des Formgebungswerkzeugs, beispielsweise die Werkzeughälften, auf- und zufährt. Formungebundenen Maßen müssen in der Regel größere Toleranzen zugestanden werden.

Insbesondere lassen sich scheibenförmige analytische Magazine erfindungsgemäß ausgestalten. Insbesondere können diese ein oder mehrere Microsampler umfassen. Weiterhin können die Testfeldflächen der Testchemien Testfelder bilden, beispielsweise mindestens ein Testfeld pro analytischem Hilfsmittel, wobei die Testfelder beispielsweise fest in dem Gehäuse des analytischen Hilfsmittels und in dem Magazingehäuse montiert sind. Das mindestens eine optionale Lanzettenelement, welches in dem analytischen Hilfsmittel enthalten sein kann, kann beweglich hierzu angeordnet sein. Eine Befestigung des Testfeldes im Gehäuse kann beispielsweise durch ein Umspritzen und/oder Hinterspritzen des Testelements während des Formgebungsverfahrens, beispielsweise während des Spritzgußverfahrens, auf einfache oder zuverlässige Weise erfolgen. Derartige Formgebungsverfahren, insbesondere Kunststoff-Formgebungsverfahren, werden bereits heute üblicherweise zur Herstellung von Gehäusen analytischer Hilfsmittel und/oder analytischer Magazine eingesetzt, so dass Standardprozesse mit lediglich geringfügiger Modifikation weiterverwendet werden können. Insbesondere können Testchemieringe als Testelemente eingesetzt werden, welche umspritzt oder hinterspritzt werden. Die oben beschriebene thermische Stabilität der vorgeschlagenen Testchemie, insbesondere bis zu einer Temperatur von 120 °C, macht sich in diesem Rahmen besonders günstig bemerkbar.

Gegenüber den aus dem Stand der Technik bekannten Verfahren, bei welchen Chemiefelder üblicherweise über mindestens einen Klebstoff mit mindestens einem Träger verbunden werden, kann im Rahmen der vorliegenden Erfindung vollständig auf Klebematerialien verzichtet werden. Insbesondere kann eine Verbindung zwischen dem Gehäuseteil und dem Testelement klebstofffrei erfolgen. Derartige Klebematerialien und Klebstoffe führen häufig zu Ausdünstungen, welche nicht nur für das Testelement und dort insbesondere die Testchemie schädlich sein können, sondern insbesondere auch eine hydrophile Beschichtung eines Microsamplers zerstören können, so dass anschließend eine Blutaufnahme oder eine andere Art der Aufnahme von Körperflüssigkeit nicht oder nur noch schwer gewährleistbar ist. Auf diese Weise kann durch die bevorzugte Klebstoff-freie Ausgestaltung auch die Qualität und Zuverlässigkeit der analytischen Hilfsmittel deutlich verbessert werden.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figur 1: Eine perspektivische Ansicht eins analytischen Magazins mit einer Mehrzahl analytischer Hilfsmittel;
- Figur 2: Eine Draufsicht auf das analytische Magazin gemäß Figur 1;
- Figur 3: Eine Schnittdarstellung durch das analytische Magazin entlang der Schnittlinie A-A in Figur 2:
- Figur 4: Eine vergrößerte Detaildarstellung des Bereichs B in Figur 3;
- Figur 5: Eine vergrößerte Detaildarstellung des Bereichs C in Figur 3;
- Figur 6: Eine perspektivische Darstellung eines Magazingehäuse-Unterteils des analytischen Magazins in den Figuren 1 bis 5;
- Figur 7: Eine vergrößerte Detaildarstellung des Bereichs A in Figur 6; und
- Figuren 8A bis 8G: verschiedene Ansichten eines Ausführungsbeispiels eines als Einzeltest ausgestalteten analytischen Hilfsmittels.

### Ausführungsbeispiele

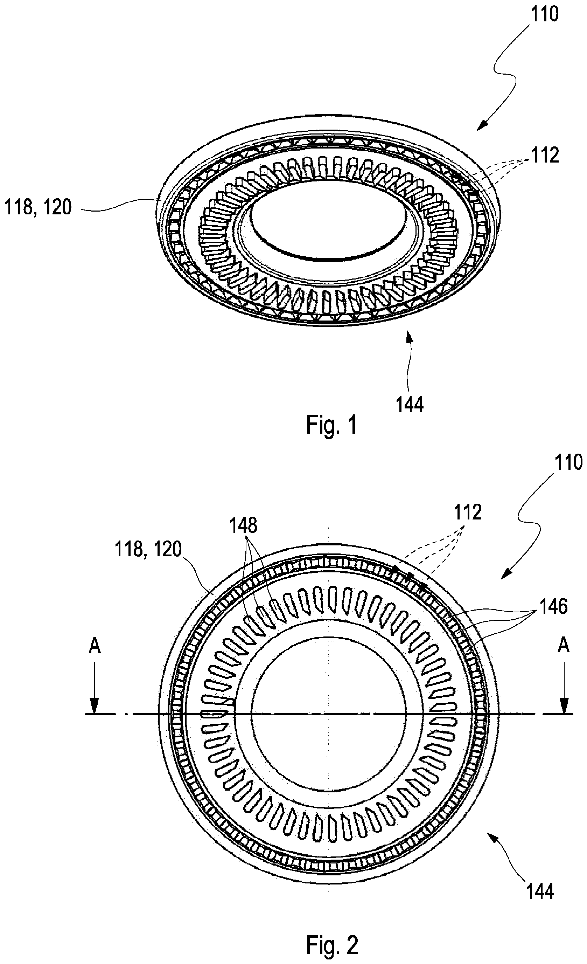

In den Figuren 1 bis 7 sind verschiedene Darstellungen eines Ausführungsbeispiels eines analytischen Magazins 110 dargestellt. Das analytische Magazin 110 ist in dem dargestellten Ausführungsbeispiel als kreisringförmiges oder kreisscheibenförmiges analytisches Magazin 110 ausgestaltet und umfasst eine Mehrzahl von analytischen Hilfsmitteln 112, die in dem analytischen Magazin 110 in einer radialen Anordnung angeordnet sind.

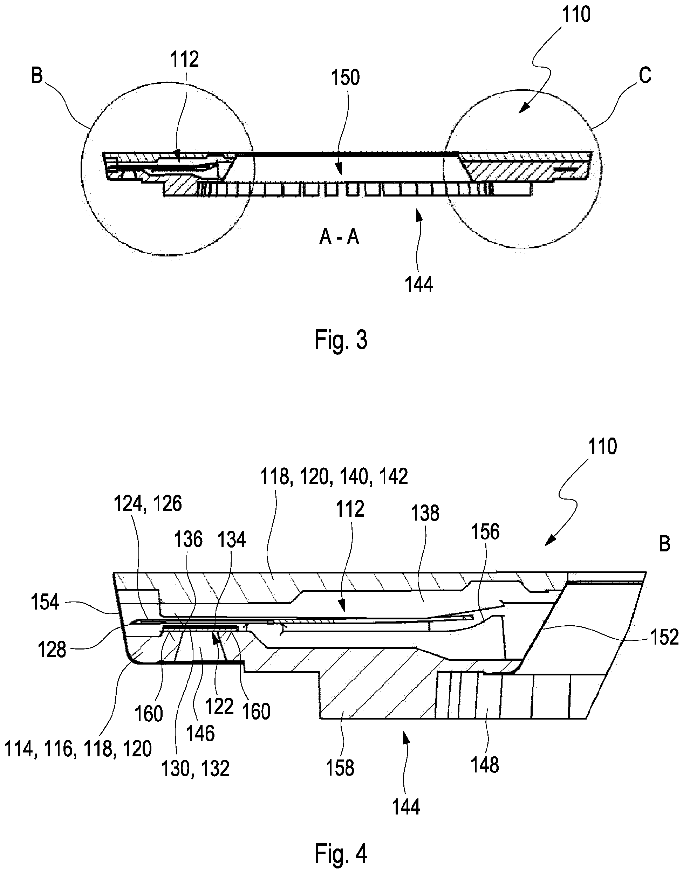

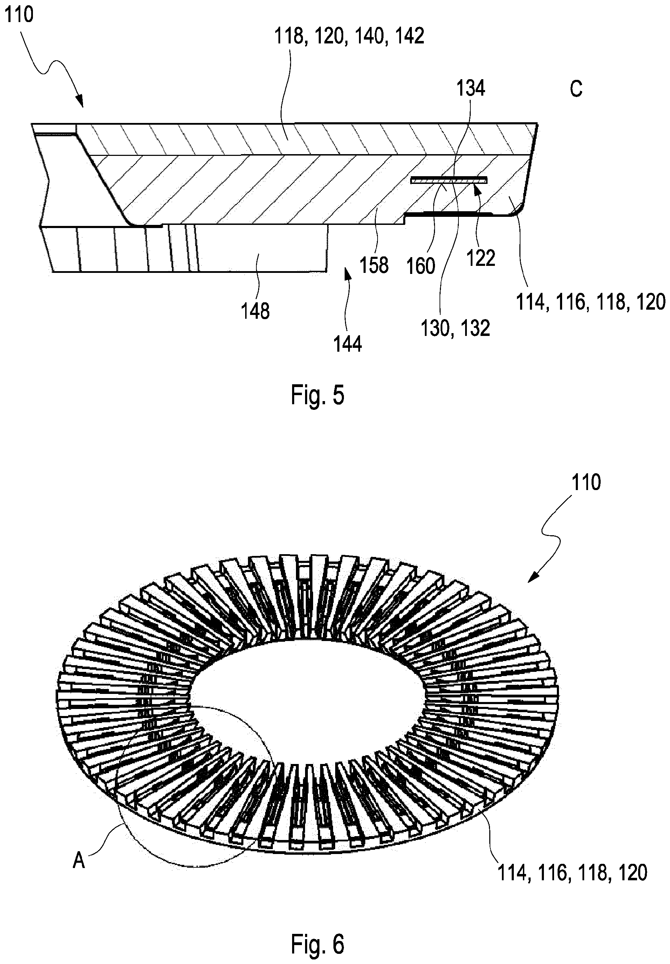

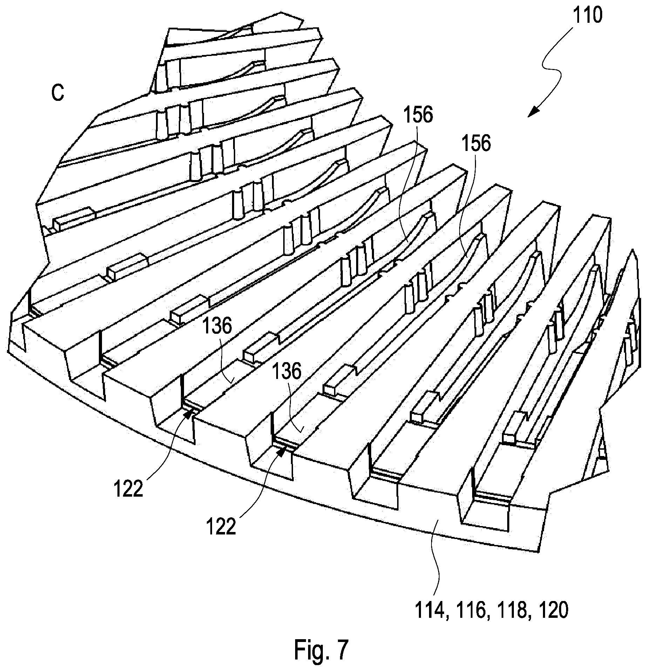

In Figur 1 ist eine perspektivische Darstellung des analytischen Magazins 110 gezeigt, in Figur 2 eine Draufsicht auf das analytische Magazin 110 auf eine Detektionsseite, und in Figur 3 ist eine Schnittdarstellung entlang einer Schnittlinie A-A in Figur 2 gezeigt. Die Figuren 4 und 5 zeigen vergrößerte Detaildarstellungen der Bereiche B bzw. C in der Schnittdarstellung gemäß Figur 3. Figur 6 zeigt eine Einzeldarstellung eines Gehäuseteils 114 des analytischen Magazins in Form eines Unterteils 116 eines Magazingehäuses 118, welches Gehäuse 120 der analytischen Hilfsmittel 112 zusammenfasst. Figur 7 zeigt eine vergrößerte Detaildarstellung des Ausschnitts A in Figur 6. Sämtliche Darstellungen werden im Folgenden gemeinsam beschrieben.

Die analytischen Hilfsmittel 112 umfassen, wie insbesondere aus der Darstellung in Figur 4 hervorgeht, in dem dargestellten Ausführungsbeispiel jeweils mindestens ein Testelement 122 und optional mindestens ein Lanzettenelement 124, welches insbesondere als Microsampler 126 ausgestaltet sein kann und welches beispielsweise auf einer in Figur 4 nach unten weisenden Seiten einen sich von einer Lanzettenspitze 128 aus erstreckenden Kapillarkanal aufweisen können, der in den Figuren nicht näher dargestellt ist. Die Testelemente 122 umfassen ein Trägerelement 130, beispielsweise eine Trägerfolie 132, auf welche eine oder mehrere Schichten einer Testchemie 134 aufgebracht sind. Die Testchemie 134 weist dem Lanzettenelement 124 zu und bildet in diesem Bereich mindestens eine Testfeldfläche 136, auf welche die flüssige Probe aufgebracht werden kann.

Bei dem gezeigten Ausführungsbeispiel ist jeweils optional mindestens eine Testfeldfläche 136 und mindestens ein Lanzettenelement 124 in einer Kammer 138 aufgenommen, welche in dem Gehäuse 120 ausgebildet ist. Ein analytisches Hilfsmittel 112 umfasst somit, neben mindestens einem Testelement 122 mit einer in der Kammer 138 angeordneten Testfeldfläche 136 und mindestens einem Lanzettenelement 124 jeweils mindestens ein Gehäuse 120, welches die Kammer 138 ausbildet. Die Gehäuse 120 der analytischen Hilfsmittel 112 des analytischen Magazins 110 sind miteinander verbunden und bilden gemeinsam Bestandteile des Magazingehäuses 118. Dabei sind in dem dargestellten Ausführungsbeispiel die Gehäuse 120 und das Magazingehäuse 118 jeweils mehrteilig ausgebildet. So umfassen die Gehäuse 120 jeweils Gehäuseteile 114, welche Bestandteile eines Unterteils 116 des Magazingehäuses 118 sind, sowie weitere Gehäuseteile 140, welche Bestandteile eines Oberteils 142 des Magazingehäuses 118 sind.

Die Kammern 138 sind, wie oben beschrieben, radial in dem kreisringförmigen analytischen Magazin 110 angeordnet. Die Figuren 6 und 7 zeigen diese radialen Anordnungen, wobei in diesen Darstellungen jeweils nur die Gehäuseteile 114 der Gehäuse 120 der analytischen Hilfsmittel 112 und das Unterteil 116 des Magazingehäuses 118 gezeigt sind. Die Lanzettenelemente 124 sind in diesen Darstellungen nicht gezeigt.

Wie beispielsweise in der Detaildarstellung gemäß Figur 4 gezeigt ist, weist das Magazingehäuse 118 auf der Unterseite in Figur 4 eine Detektionsseite 144 auf, auf welcher in dem Unterteil 116 und in den Gehäuseteilen 114 jeweils Sichtfenster 146 in Form von Öffnungen ausgebildet sind. Diese Sichtfenster 146 sind auch in der Draufsicht auf die Detektionsseite 144 in Figur 2 erkennbar. Durch diese Sichtfenster 146 hindurch sind die Trägerelemente 130 der Testelemente 122 erkennbar. Diese Trägerelemente 130 sind, wie unten noch näher ausgeführt wird, vorzugsweise transparent ausgestaltet, so dass eine optische Detektion einer optischen Eigenschaftsänderung der Testchemie 134 des jeweiligen Testelements 122 durch die Sichtfenster 146 hindurch beobachtbar ist.

Das analytische Magazin 110 weist, beispielsweise auf der Detektionsseite 144, vorzugsweise eine Mehrzahl von Transportelementen 148 auf, mittels derer durch ein Testgerät, welches das analytische Magazin 110 verwendet, jeweils genau ein analytisches Hilfsmittel 112 in eine Applikationsposition gebracht werden kann. In dieser Applikationsposition kann beispielsweise ein in den Figuren nicht dargestellter Aktor durch eine zentrale Aussparung 150 in dem analytischen Magazin 110 (siehe Figur 3) durch eine proximale Öffnung 152, auch als Aktoröffnung zu bezeichnen, in die Kammer 138 eingreifen. Dort kann der Aktor beispielsweise ein proximales Ende des Lanzettenelements 124 ergreifen, beispielsweise eine dort angeordnete Öse und/oder eine andere Art des Verbindungselements. Dann kann der Aktor das Lanzettenelement 124 zu einer radial nach außen gerichteten Lanzettenbewegung (in Figur 4 nach links gerichtet) antreiben, wobei die Lanzettenspitze 128 die Kammer 138 durch eine distale Öffnung 154 verlässt, einen Einstich oder Einschnitt in eine Haut eines Benutzers vornimmt und eine flüssige Probe aufnimmt, beispielsweise mit dem Kapillarelement. Anschließend kann der Aktor eingerichtet sein, um eine Remagazinierung des Lanzettenelements 124 durchzuführen, beispielsweise indem das Lanzettenelement 124 wieder zurück in die Kammer 138 gezogen wird, in welcher das Lanzettenelement 124 zuvor bereits gelagert war.

Anschließend kann ein Probenübertrag auf die Testfeldfläche 136 des Testelements 122 erfolgen. Dies kann beispielsweise dadurch erfolgen, dass das Lanzettenelement, beispielsweise durch eine entsprechende Gestaltung der Wände der Kammer 138, kurzfristig stark an die Testfeldfläche 136 angenähert wird. Beispielsweise können zu diesem Zweck eine oder mehrere entsprechende Konturen 156 in dem Gehäuseteil 114 vorgesehen sein, wie beispielsweise in den Figuren 4 und 7 erkennbar ist. Auf diese Weise kann beispielsweise bei einer Remagazinierung ein Übertrag der flüssigen Probe auf die Testfeldfläche 136 des jeweiligen analytischen Hilfsmittels 112 erfolgen.

Es wird, wie oben ausgeführt, das Testelement 122 während eins Formgebungsverfahrens und vorzugsweise auch durch dieses Formgebungsverfahren mit dem Gehäuseteil 114 verbunden. Bei dem analytischen Magazin 110 gemäß dem dargestellten Ausführungsbeispiel erfolgt dies vorzugsweise für alle analytischen Hilfsmittel 112 des analytischen Magazins 110 gleichzeitig. Auch andere Ausgestaltungen sind jedoch grundsätzlich denkbar. Zu diesem Zweck kann beispielsweise, wie in dem dargestellten Ausführungsbeispiel, ein Trägerelement 130 in Form einer kreisringförmigen Trägerfolie 132 verwendet werden. Diese Trägerfolie 132 ist mit der Testchemie 134 beschichtet. Das Trägerelement 130 wird in ein Formgebungswerkzeug, beispielsweise ein Spritzgießwerkzeug, mittels dessen das Unterteil 116 und damit die Gehäuseteile 114 herstellbar sind, eingelegt. Das Formgebungswerkzeug ist derart geformt, dass die Testchemie 134 in dem Bereich der späteren Testfeldflächen 136 auf einer Werkzeugwand des Formgebungswerkzeugs aufliegt, so dass bei dem Formgebungsprozess die Testfeldflächen 136 nicht mit Gehäusewerkstoff 158 des Gehäuseteils 114 bzw. des Unterteils 116 bedeckt werden. In anderen Bereichen hingegen, in welchen später keine Testfeldfläche 136 entstehen soll, ist die Testchemie 134 von der Wand des Formgebungswerkzeugs beabstandet, so dass diese Bereiche in das Gehäusewerkstoff 158 eingebettet werden. Dies ist beispielsweise in Figur 5 erkennbar, welche einen Schnitt durch einen Wandabschnitt des Unterteils 116 zwischen zwei Kammern 138 zeigt. In diesem Bereich ist auch die Testchemie 134 in das Gehäusewerkstoff 158 eingebettet.

Nach diesem Einlegen des mit der Testchemie 134 beschichteten Trägerelements 130 in Form eines Testchemierings in das Formgebungswerkzeug wird das Gehäusewerkstoff 158 in dieses Formgebungswerkzeug eingebracht, beispielsweise durch Einspritzen oder Einpressen, wobei sich das Gehäusewerkstoff 158 in einem flüssigen oder zumindest verformbaren Zustand befindet. Bei diesem Verfahrensschritt und/oder einem nachfolgenden Erstarrungsschritt, wobei diese Schritte vorzugsweise in dem Werkzeug durchgeführt werden, wird das Gehäuseteil 114 an mindestens einer Fläche 160 mit dem Trägerelement 130 direkt verbunden. Dies kann beispielsweise dadurch begünstigt werden, dass das Trägerelement 130 und das Gehäusewerkstoff 158 chemisch ähnlich ausgestaltet werden, beispielsweise indem Polycarbonate und/oder Polyester verwendet werden.

Der Testchemiering, welcher Trägerelemente 130 und Testchemie 134 für die analytischen Hilfsmittel 112 bereitstellt, wird somit bei diesem Formgebungsverfahren vorzugsweise hinterspritzt (beispielsweise in dem in Figur 4 dargestellten Bereich) und/oder umspritzt (beispielsweise in dem in Figur 5 dargestellten Bereich. Im Bereich der Testfeldflächen 136, in welchen vorzugsweise die Testchemie 134 auf einer Werkzeugwand des Formgebungswerkzeugs aufliegt, können dabei Temperaturen während des Formgebungsverfahrens eingestellt werden, welche beispielsweise 120 °C nicht überschreiten. Durch eine geeignete Auswahl der Testchemie 134, welche diesen Temperaturen zumindest kurzfristig Stand hält, ist ein derartiger Temperaturanstieg noch akzeptabel. Das Sichtfenster 146 kann beispielsweise kleiner dimensioniert werden als die Testfeldfläche 136, so dass beispielsweise Randbereiche der Testfeldfläche 136, an welchen die Testchemie 134 während des Formgebungsverfahrens mit dem heißen Gehäusewerkstoff 158 in Kontakt gerät, bei der optischen Auswertung nicht berücksichtigt werden. Beispielsweise kann das Sichtfenster 146 in jeder Dimension um mindestens 5 % kleiner dimensioniert werden, als die Testfeldfläche 136, insbesondere um mindestens 10 % und beispielsweise um mindestens 30 %, beispielsweise 30 bis 50 % kleiner.

Durch den beschriebenen Formgebungsprozess kann auf einfache, zuverlässige und großtechnisch leicht umsetzbare Weise ein Verbinden der Testelemente 122 der analytischen Hilfsmittel 112, vorzugsweise sämtlicher analytischer Hilfsmittel 112, mit den Gehäuseteilen 114 erfolgen, ohne dass beispielsweise ein separater Auftrag von Klebstoff auf die Gehäuseteile 114 und/oder die Testelemente 112 erforderlich wäre, um die Verbindung zwischen diesen Elementen herzustellen. Es lassen sich somit Verfahrensschritte einsparen. Gleichzeitig lassen sich, wie oben beschrieben, durch den beschriebenen Aspekt analytische Hilfsmittel 112 und analytische Magazine 110 mit geringen Fertigungstoleranzen herstellen, da beispielsweise Fertigungstoleranzen eines Klebstoffs vermieden werden können.

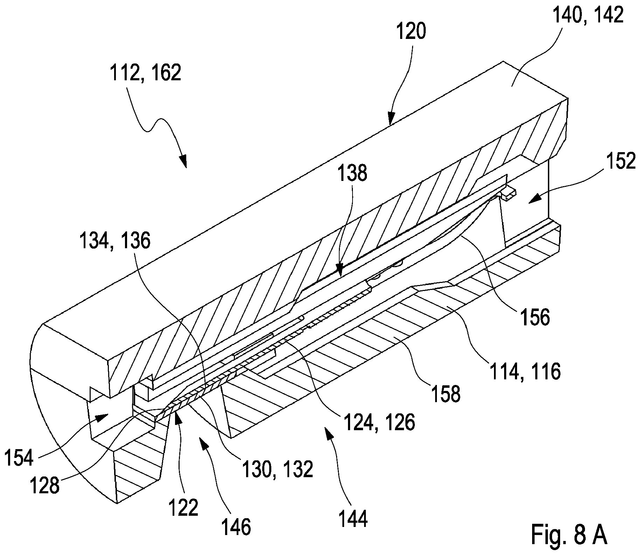

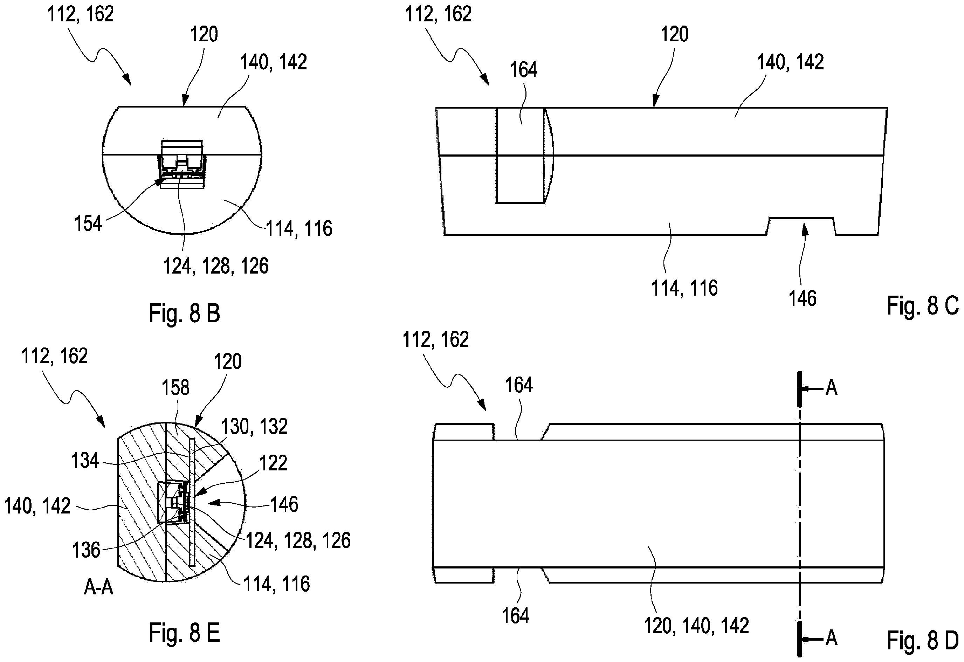

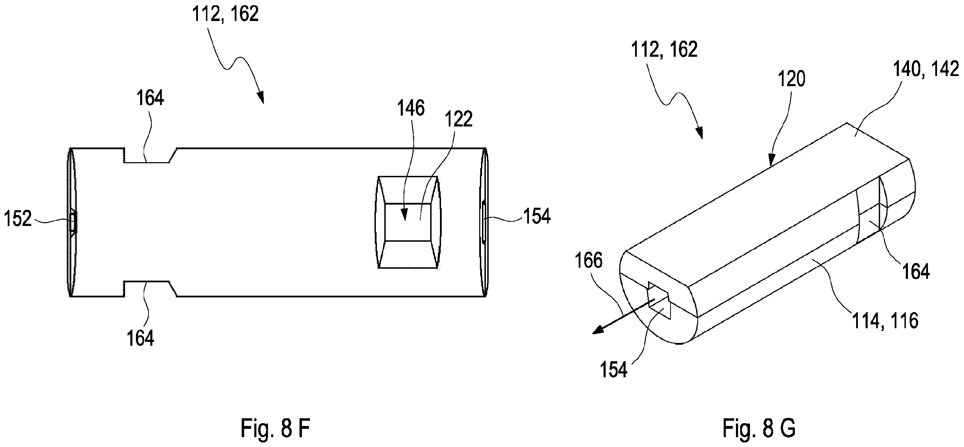

In den Figuren 8A-8G ist in verschiedenen Ansichten ein als Einzeltest 162 ausgebildetes analytisches Hilfsmittel 112 dargestellt. Dabei zeigt Figur 8A eine Schnittdarstellung durch den Einzeltest 162 entlang einer Achse einer Kammer 138 des Einzeltests 162, Figur 8B zeigt eine Vorderansicht des Einzeltests 162 mit Blickrichtung auf eine distale Öffnung 154 zum Austritt einer Lanzettenspitze 128 eines Lanzettenelements 124, Figur 8C eine Seitenansicht des Einzeltests 162, Figur 8D eine Ansicht des Einzeltests 162 mit Blickrichtung in Figur 8C von oben, Figur 8E eine Schnittdarstellung entlang der Schnittlinie A-A in Figur 8D, Figur 8F eine Ansicht des Einzeltests 162 mit Blickrichtung in Figur 8C von unten, und Figur 8D eine perspektivische Darstellung des Einzeltests 162. Die Figuren werden im Folgenden gemeinsam erläutert.

Der Einzeltest 162 umfasst wiederum, wie in Figur 8A dargestellt, ein Gehäuse 120, welche mehrteilig ausgebildet ist. Dieses Gehäuse 120 kann beispielsweise, wie in Figur 8A erkennbar, ein Gehäuseteil 114 in Form eines Unterteils 116 und ein weiteres Gehäuseteil 140 als Oberteil 142 aufweisen. Das Gehäuse 120 kann eine Kammer 138 umschließen, in welcher optional mindestens ein Lanzettenelement 124 aufgenommen sein kann. Dieses Lanzettenelement 124 kann beispielsweise mittels entsprechender Konturen 156 gekrümmt gelagert sein, sodass, wie auch in den vorhergehenden Ausführungsbeispielen, das Lanzettenelement 124 durch seine eigene Federkraft in der Kammer 138 gehalten werden kann.

Auf diese Weise kann beispielsweise auch nach einem Stechvorgang, wie oben ausgeführt, eine sichere Remagazinierung des Lanzettenelements 124 in der Kammer 138 gewährleistet werden. Das Lanzettenelement 124 kann beispielsweise wiederum als Microsampler 126 ausgestaltet sein.

Die Kammer 138 kann beispielsweise mindestens eine Austrittsöffnung für eine Lanzettenspitze 128 der Lanzette 124 aufweisen. Diese Austrittsöffnung ist hier als distale Öffnung 154 bezeichnet. Optional kann die Kammer 138 weiterhin mindestens eine Aktoröffnung oder proximale Öffnung 152 aufweisen. Diesbezüglich kann wiederum auf die obige Beschreibung des analytischen Magazins verwiesen werden.

Weiterhin umfasst das analytische Hilfsmittel wiederum ein Testelement 122 mit optional einem Trägerelement 130 und einer dem Innenraum der Kammer 138 zuweisenden Testchemie 134, welche auf der der Kammer 138 zuweisenden Seite eine Testfeldfläche 136 bildet. Diesbezüglich kann beispielsweise auf die Beschreibung der Figur 4 oben verwiesen werden.

Der Einzeltest 162 kann grundsätzlich analog zu den oben beschriebenen Herstellungsverfahren hergestellt werden. Zu diesem Zweck kann beispielsweise das fertige Testelement 162 in ein entsprechendes Werkzeug eingelegt werden, sodass beispielsweise die Testfeldfläche 136 vollständig oder teilweise auf einer Wand des Werkzeugs aufliegt. Anschließend kann ein Gehäusematerial, welches das Gehäuseteil 114 bildet, in das Werkzeug eingebracht werden, beispielsweise eingespritzt werden, sodass durch den Formgebungsprozess das Trägerelement 130 mit dem Gehäusematerial verbunden wird und gleichzeitig das Gehäuseteil 114 gebildet wird. Dabei kann beispielsweise das Trägerelement 130 stoffschlüssig und ohne Zwischenschaltung weiterer Verbindungselemente oder Verbindungsmaterialien mit dem Gehäuseteil 114 verbunden werden. Diesbezüglich kann exemplarisch wiederum auf die obige Beschreibung des analytischen Magazins 110 verwiesen werden.

Der Einzeltest 162 kann beispielsweise weiterhin wiederum auf einer Detektionsseite 144 ein Sichtfenster 146 aufweisen. Durch dieses Sichtfenster 146 kann, analog zu den oben beschriebenen Ausführungsbeispielen, beispielsweise eine Veränderung mindestens einer optischen Eigenschaft des Testelements 122 bzw. der Testchemie 134 bei einer Reaktion mit der Probe und/oder einem in der Probe enthaltenen Analyten detektiert werden.

Das Trägerelement kann beispielsweise wiederum als Trägerfolie 132 ausgestaltet sein. Bezüglich möglicher Werkstoffe kann auf die obige Beschreibung verwiesen werden. Beipsielsweise können Polycarbonate und/oder andere Kunststoffwerkstoffe eingesetzt werden, vorzugsweise transparente Werkstoffe. Wie beispielsweise aus der Schnittdarstellung in Figur 8E hervorgeht, kann die Testchemie 134 außerhalb der Testfeldfläche 136, welche von der Kammer 138 aus zugänglich ist, in den Gehäusewerkstoff 158 des Gehäuseteils 114 eingebettet sein. Die Beschichtung des Trägerelements 130 mit der Testchemie 134 kann somit vorzugsweise vor dem Formgebungsprozess erfolgen, sodass die Testchemie 134 teilweise in den Gehäusewerkstoff 158 eingebettet ist. Für weitere Einzelheiten kann auf die obige Beschreibung verwiesen werden.

Der Einzeltest 162 kann einzeln gehandhabt werden, beispielsweise indem dieser Einzeltest 162 einzeln von einem (nicht dargestellten) Aktor greifbar und verwendbar ist. Beispielsweise kann der Einzeltest 162 optional allgeine gelagert sein oder kann optional mit mehreren anderen Einzeltests 162 in einem Magazin gelagert und einzeln dem Magazin entnehmbar sein, um einzeln verwendet zu werden. Der Einzeltest 162 ist somit in dem dargestellten Ausführungsbeispiel nicht mit anderen Einzeltests 162 unmittelbar mechanisch verbunden, sondern kann als einzelnes Element verwendet und gehandhabt werden. Beispielsweise kann der Einzeltest 162 unabhängig von anderen Einzeltests mittels einer entsprechenden Aktorik einem analytischen Testgerät zugeführt werden und/oder einer Applikationsposition innerhalb des analytischen Testgeräts zugeführt werden, um dort verwendet zu werden. In dieser Applikationsposition kann beispielsweise ein entsprechender Aktor durch die proximale Öffnung 152 in die Kammer 138 eingreifen, das Lanzettenelement 124 an einem der proximalen Öffnung 152 zuweisenden Ende ergreifen, eine Stechbewegung durchführen, wobei die Lanzettenspitze 128 durch die distale Öffnung 154 kurzfristig austritt und in eine Hautoberfläche einsticht. Dabei wird Blut und/oder andere Körperflüssigkeit aufgenommen, und die Lanzettenspitze 128 wird wieder zurückgezogen in die Kammer 138. Dabei wird durch die Konturen 156 und/oder einen separaten Aktor die Lanzettenspitze 128 derart dicht an der Testfeldfläche 136 vorbeigeführt, dass aufgenommene Probe auf die Testfeldfläche 136 im Inneren der Kammer 138 übertragen wird.

Das Gehäuse 120 des Einzeltests 162 kann eine oder mehrere Gehäusekonturen und/oder Haltestrukturen aufweisen, welche eine Handhabung des Einzeltests 162 mittels eines entsprechenden Aktors erleichtern können. Beispielsweise können an einer Seitenwand des Gehäuses 120 eine oder mehrere Nuten 164 vorgesehen sein, welche beispielsweise in den Figuren 8C, 8D, 8F und 8G erkennbar sind. Alternativ oder zusätzlich zu den Nuten 164 kann der Einzeltest 162 optional auch eine oder mehrere auf andere Weise gestaltete Haltestrukturen aufweisen, sodass allgemein der Einzeltest 162 vorzugsweise ein oder mehrere derartiger Haltestrukturen aufweisen kann, die ein Zusammenwirken mit einer Aktorik zur mechanischen Handhabung der Einzeltests 162 ermöglichen und/oder erleichtern.

Alternativ oder zusätzlich können als Haltestruktur auch andere Arten von Verbindungselementen vorgesehen sein. Weiterhin ist in der perspektivischen Darstellung gemäß Figur 8G auch symbolisch eine Stechrichtung 166 dargestellt, also eine Richtung, in welcher die in Figur 8A erkennbare Lanzettenspitze 128 aus der distalen Öffnung 154 austritt, um in eine Hautpartie eines Benutzers einzudringen, um, anschließend, entgegen der Stechrichtung 166 wieder zurück in die Kammer 138 zurückgeführt zu werden.

### Bezugszeichenliste

- 110: analytisches Magazin
- 112: analytisches Hilfsmittel
- 114: Gehäuseteil
- 116: Unterteil
- 118: Magazingehäuse
- 120: Gehäuse
- 122: Testelement
- 124: Lanzettenelement
- 126: Microsampler
- 128: Lanzettenspitze
- 130: Trägerelement
- 132: Trägerfolie
- 134: Testchemie
- 136: Testfeldfläche
- 138: Kammer
- 140: weiteres Gehäuseteil
- 142: Oberteil
- 144: Detektionsseite
- 146: Sichtfenster
- 148: Transportelement
- 150: zentrale Aussparung
- 152: proximale Öffnung, Aktoröffhung
- 154: distale Öffnung
- 156: Konturen
- 158: Gehäusewerkstoff
- 160: Fläche
- 162: Einzeltest
- 164: Nut
- 166: Stechrichtung

## Patentansprüche

1. Verfahren zum Herstellen mehrerer analytischer Hilfsmittel (112) zum Nachweis mindestens eines Analyten in einer Probe, insbesondere einer Körperflüssigkeit, wobei die analytischen Hilfsmittel (112) jeweils mindestens ein Gehäuse (120) mit einem Gehäuseteil (114) und mindestens ein Testelement (122) mit mindestens einer Testchemie (134) aufweisen, wobei das Verfahren folgende Schritte umfasst:
a) Bereitstellen der Testelemente (122); und
b) Herstellen der Gehäuseteile (114) mittels mindestens eines Formgebungsverfahrens, wobei mittels des Formgebungsverfahrens die Testelemente (122) mit den jeweiligen Gehäuseteilen (114) verbunden werden,
wobei das Verfahren derart durchgeführt wird, dass die analytischen Hilfsmittel (112) in einem analytischen Magazin (110) enthalten sind, wobei die analytischen Hilfsmittel (112) in einem gemeinsamen Magazingehäuse (118) aufgenommen werden, wobei das Verfahren derart durchgeführt wird, dass die Gehäuse (120) der analytischen Hilfsmittel (112) Bestandteile des Magazingehäuses (118) sind, und wobei die analytischen Hilfsmittel (112) des Magazins (110) dadurch hergestellt werden, dass bei dem Formgebungsverfahren die Gehäuseteile (114) der analytischen Hilfsmittel (112) gleichzeitig hergestellt und gleichzeitig miteinander verbunden werden.

2. Verfahren nach dem vorhergehenden Anspruch, wobei das Formgebungsverfahren mindestens ein Gießverfahren umfasst.

3. Verfahren nach einem der vorhergehenden Ansprüche, wobei das Formgebungsverfahren mindestens ein Kunststoff-Formgebungsverfahren umfasst, wobei das Kunststoff-Formgebungsverfahren ausgewählt ist aus einem Hinterspritzen und einem Umspritzen.

4. Verfahren nach einem der vorhergehenden Ansprüche, wobei in Verfahrensschritt b) von jedem der Testelemente (122) jeweils mindestens ein Teil in ein Formgebungswerkzeug eingelegt wird und in dem Formgebungswerkzeug zumindest teilweise mit mindestens einem Gehäusewerkstoff (158) des jeweiligen Gehäuseteils (114) in Kontakt gebracht wird, wobei der mindestens eine in das Formgebungswerkzeug eingelegte Teil jeweils mit mindestens einem Abschnitt (136) auf mindestens einer Wand des Formgebungswerkzeugs aufliegt, wobei der Verfahrensschritt b) derart durchgeführt wird, dass die Wand des Formgebungswerkzeugs, zumindest in den Bereichen, auf welchen der jeweilige Abschnitt (136) auf der Wand aufliegt, während der Durchführung des Verfahrensschritts b) eine Temperatur von nicht mehr als 130 °C, vorzugsweise von nicht mehr als 120 °C und besonders bevorzugt von nicht mehr als 110 °C aufweist.

5. Verfahren nach dem vorhergehenden Anspruch, wobei jeweils der Abschnitt (136), welcher auf der Wand aufliegt, zumindest einen Teil der Testchemie (134) umfasst, insbesondere eine Testfeldfläche (136) der Testchemie (134).

6. Verfahren nach einem der vorhergehenden Ansprüche, wobei im Verfahrensschritt b) die Testelemente (122) jeweils derart mit dem Gehäuseteil (114) verbunden werden, dass jeweils mindestens eine Oberfläche der Testchemie (134) als Testfeldfläche (136) für eine Aufgabe der Probe von einem Gehäusewerkstoff (158) des Gehäuseteils (114) unbedeckt verbleibt, wobei das Gehäuse (120) derart ausgestaltet wird, dass mindestens ein Sichtfenster (146) in dem Gehäuse (120) entsteht, wobei die Testfeldfläche (136) durch das Sichtfenster (146) des Gehäuses (120) optisch überwachbar ist.

7. Verfahren nach dem vorhergehenden Anspruch, wobei das Verfahren derart durchgeführt wird, dass das Gehäuse (120) jeweils mindestens eine Kammer (138) bildet, wobei die Testfeldfläche (136) einem Innenraum der Kammer (138) zuweist.

8. Verfahren nach einem der vorhergehenden Ansprüche, wobei die Testchemie (134) derart stabil ausgewählt wird, dass diese zumindest kurzzeitig stabil gegenüber Temperaturen von 100 °C ist, insbesondere gegenüber Temperaturen von 110 °C und besonders bevorzugt gegenüber Temperaturen von 120 °C.

9. Verfahren nach einem der vorhergehenden Ansprüche, wobei die Testelemente (122) jeweils mindestens ein Trägerelement (130) aufweisen, wobei die Testchemie (134) mit dem Trägerelement (130) verbunden ist, insbesondere durch Aufbringen mindestens einer Schicht der Testchemie (134) auf das Trägerelement (130).

10. Verfahren nach dem vorhergehenden Anspruch, wobei das Trägerelement (130) jeweils als scheibenförmiges Trägerelement (130) ausgestaltet wird, insbesondere als kreisscheibenförmiges Trägerelement (130) und besonders bevorzugt als kreisringförmiges Trägerelement (130), wobei die Testchemie (134) als Beschichtung auf das Trägerelement (130) aufgebracht wird, wobei die Beschichtung Testfeldflächen (136) für die analytischen Hilfsmittel (112) bereitstellt.

11. Analytisches Magazin (110), umfassend eine Mehrzahl analytischer Hilfsmittel (112) zum Nachweis mindestens eines Analyten in einer Probe, wobei die analytischen Hilfsmittel (112) jeweils aufweisen:
- mindestens ein Gehäuse (120) mit mindestens einem Gehäuseteil (114) und
- mindestens ein Testelement (122) mit mindestens einer Testchemie (134),
wobei die analytischen Hilfsmittel (112) in einem gemeinsamen Magazingehäuse (118) aufgenommen sind, wobei die Gehäuse (120) der analytischen Hilfsmittel (112) Bestandteile des Magazingehäuses (118) sind, wobei die Gehäuseteile (114) mittels eines Formgebungsverfahrens hergestellt und mittels des Formgebungsverfahrens mit dem jeweiligen Testelement (122) verbunden werden, wobei bei dem Formgebungsverfahren die Gehäuseteile (114) der analytischen Hilfsmittel (112) gleichzeitig hergestellt und gleichzeitig miteinander verbunden werden.

12. Analytisches Magazin (110) nach dem vorhergehenden Anspruch, wobei die analytischen Hilfsmittel (112) weiterhin jeweils mindestens ein Lanzettenelement zur Generierung der Probe aufweisen, insbesondere mindestens einen Microsampler.

13. Analytisches Magazin (110) nach einem der Ansprüche 11 oder 12, wobei die Gehäuse (120) jeweils mindestens ein von einer Außenseite des Gehäuses (120) zugängliches Sichtfenster (146) aufweisen, wobei mindestens eine Eigenschaftsänderung des jeweiligen Testelements (122), insbesondere mindestens eine Farbänderung und/oder mindestens eine Änderung mindestens einer optischen Eigenschaft, durch das Sichtfenster (146) von außen detektierbar ist.

14. Analytisches Magazin (110) nach dem vorhergehenden Anspruch, wobei das Sichtfenster (146) derart ausgestaltet ist, dass mindestens eine Testfeldfläche (136) des jeweiligen Testelements (122) durch mindestens ein optisch zumindest teilweise transparentes Trägerelement (130) des jeweiligen Testelements (122) hindurch beobachtbar ist.

## Claims

1. Method for producing a plurality of analytical aids (112) for the detection of at least one analyte in a sample, in particular, a body fluid, wherein the analytical aids (112) each comprise at least one housing (120) comprising a housing part (114) and at least one test element (122) comprising at least one test chemistry (134), wherein the method comprises the following steps:
a) providing the test elements (122); and
b) producing the housing parts (114) by means of at least one shaping process, wherein the test elements (122) are connected to the respective housing parts (114) by means of the shaping process,
wherein the method is carried out such that the analytical aids (112) are contained in an analytical magazine (110), wherein the analytical aids (112) are accommodated in a common magazine housing (118), wherein the method is carried out such that the housings (120) of the analytical aids (112) are constituents of the magazine housing (118) and wherein the analytical aids (112) of the magazine (110) are produced by the housing parts (114) of the analytical aids (112) being simultaneously produced and simultaneously connected to one another during the shaping process.

2. Method according to the preceding claim, wherein the shaping process comprises at least one casting process.

3. Method according to any one of the preceding claims, wherein the shaping process comprises at least one plastics shaping process, wherein the plastics shaping process is selected from in-mould coating and overmoulding.

4. Method according to any one of the preceding claims, wherein, in method step b), of each of the test elements (122), in each case at least one part is inserted into a shaping mould and is at least partly contacted, in the shaping mould, with at least one housing material (158) of the respective housing part (114), wherein the at least one part inserted into the shaping mould rests, in each case via at least one section (136), on at least one wall of the shaping mould, wherein method step b) is carried out such that the wall of the shaping mould, at least in the regions on which the respective section (136) rests on the wall, has a temperature of not more than 130°C, preferably not more than 120°C and more preferably not more than 110°C while method step b) is being carried out.

5. Method according to the preceding claim, wherein in each case the section (136) which rests on the wall comprises at least part of the test chemistry (134), in particular a test field area (136) of the test chemistry (134).

6. Method according to any one of the preceding claims, wherein, in method step b), the test elements (122) are connected in each case to the housing part (114) such that in each case at least one surface of the test chemistry (134) as test field area (136) for input of the sample remains uncovered by a housing material (158) of the housing part (114), wherein the housing (120) is configured such that at least one observation window (146) is produced in the housing (120), wherein the test field area (136) is optically monitorable through the observation window (146) of the housing (120).

7. Method according to the preceding claim, wherein the method is carried out such that the housing (120) forms in each case at least one chamber (138), wherein the test field area (136) faces an interior space of the chamber (138).

8. Method according to any one of the preceding claims, wherein the test chemistry (134) is selected in terms of stability such that it is stable, at least for a short time, with respect to temperatures of 100°C, in particular with respect to temperatures of 110°C and in particular preferably with respect to temperatures of 120°C.

9. Method according to any one of the preceding claims, wherein the test elements (122) each comprise at least one support element (130), wherein the test chemistry (134) is connected to the support element (130), in particular by applying at least one layer of the test chemistry (134) to the support element (130).

10. Method according to the preceding claim, wherein the support element (130) in each case is a disc-shaped support element (130), in particular a circular-disc-shaped support element (130) and particularly preferably a circular-ring-shaped support element (130), wherein the test chemistry (134) is applied to the support element (130) as a coating, wherein the coating provides test field areas (136) for the analytical aids (112).

11. Analytical magazine (110) comprising a plurality of analytical aids (112) for the detection of at least one analyte in a sample, wherein the analytical aids (112) each comprise:
- at least one housing (120) comprising at least one housing part (114) and
- at least one test element (122) comprising at least one test chemistry (134),
wherein the analytical aids (112) are accommodated in a common magazine housing (118), wherein the housings (120) of the analytical aids (112) are constituents of the magazine housing (118), wherein the housing parts (114) are produced by means of a shaping process and connected to the respective test element (122) by means of the shaping process, wherein the housing parts (114) of the analytical aids (112) are simultaneously produced and simultaneously connected to one another during the shaping process.

12. Analytical magazine (110) according to the preceding claim, wherein the analytical aids (112) each comprise in addition at least one lancet element for generating the sample, in particular at least one microsampler.

13. Analytical magazine (110) according to any one of Claims 11 and 12, wherein the housings (120) each comprise at least one observation window (146) accessible from an outer side of the housing (120), wherein at least one property change of the respective test element (122), in particular at least one colour change and/or at least one change in at least one optical property, is detectable from outside through the observation window (146).

14. Analytical magazine (110) according to the preceding claim, wherein the observation window (146) is configured such that at least one test field area (136) of the respective test element (122) is observable through at least one support element (130), which is at least partially optically transparent, of the respective test element (122).

## Revendications

1. Procédé destiné à fabriquer plusieurs outils d'analyse (112) pour détecter la présence d'au moins un analyte dans un échantillon, notamment un fluide corporel, les outils d'analyse (112) comportant chacun au moins un boîtier (120) avec une partie de boîtier (114) et au moins un élément de test (122) avec au moins une substance chimique (134) de test, le procédé comprenant les étapes suivantes :
a) mise à disposition des éléments de test (122) ; et
b) fabrication des parties de boîtier (114) au moyen d'au moins un procédé de façonnage, alors qu'au moyen du procédé de façonnage, on relie les éléments de test (122) avec les parties de boîtier respectives (114),
le procédé étant réalisé de telle sorte que les outils d'analyse (112) soient contenus dans un magasin d'analyse (110), les outils d'analyse (112) étant réceptionnés dans un boîtier formant un magasin (118) commun, le procédé étant réalisé de telle sorte que les boîtiers (120) des outils d'analyse (112) soient des composants du boîtier formant un magasin (118) et les outils d'analyse (112) du magasin (110) étant fabriqués de telle sorte que lors du procédé de façonnage, les parties de boîtier (114) des outils d'analyse (112) soient fabriquées simultanément et soient assemblées les unes aux autres simultanément.

2. Procédé selon la revendication précédente, le procédé de façonnage comprenant au moins un procédé de coulée.

3. Procédé selon l'une quelconque des revendications précédentes, le procédé de façonnage comprenant au moins un procédé de façonnage de matière plastique, le procédé de façonnage de matière plastique étant choisi parmi une rétro-injection et un enrobage par injection.

4. Procédé selon l'une quelconque des revendications précédentes, lors duquel, dans l'étape de procédé b), on insère au moins une partie de chacun des éléments de test (122) dans un outil de façonnage et dans l'outil de façonnage, on la met en contact au moins en partie avec au moins une matière (158) de boîtier de la partie de boîtier respective (114), l'au moins une partie insérée dans l'outil de façonnage reposant par au moins une section (136), sur au moins une paroi de l'outil de façonnage, l'étape de procédé b) étant réalisée de telle sorte qu'au moins dans les régions dans lesquelles la section respective (136) repose sur la paroi, pendant la réalisation de l'étape de procédé b), la paroi de l'outil de façonnage présente une température qui n'est pas supérieure à 130°C, de préférence pas supérieure à 120°C et de manière particulièrement préférée, pas supérieure à 110°C.

5. Procédé selon la revendication précédente, la section (136) qui repose sur la paroi comprenant à chaque fois au moins une partie de la substance chimique (134) de test, notamment une surface de zone de test (136) de la substance chimique (134) de test.

6. Procède selon l'une quelconque des revendications précédentes, dans lequel, pendant l'étape de procédé b), on relie à chaque fois les éléments de test (122) avec la partie de boîtier (114) de telle sorte qu'à chaque fois au moins une surface de la substance chimique (134) de test reste découverte par une matière (158) de boîtier de la partie de boîtier (114), en tant que surface de zone de test (136) pour une application de l'échantillon, le boîtier (120) étant conçu de telle sorte qu'au moins un regard (146) soit ménagé dans le boîtier (120), la surface de zone de test (136) pouvant être visuellement surveillée à travers le regard (146) du boîtier (120).

7. Procédé selon la revendication précédente, le procédé étant réalisé de telle sorte que le boîtier (120) forme à chaque fois au moins un compartiment (138), la surface de zone de test (136) étant dirigée vers un espace intérieur du compartiment (138).

8. Procédé selon l'une quelconque des revendications précédentes, la substance chimique (134) de test étant choisie d'une stabilité telle qu'elle soit au moins brièvement stable à des températures de 100°C, notamment à des températures de 110°C et de manière particulièrement préférée à des températures de 120°C.

9. Procédé selon l'une quelconque des revendications précédentes, les éléments de test (122) comportant chacun au moins un élément porteur (130), la substance chimique (134) de test étant reliée avec l'élément porteur (130), notamment par application d'au moins une couche de la substance chimique (134) de test sur l'élément porteur (130).

10. Procédé selon la revendication précédente, l'élément porteur (130) étant conçu à chaque fois en tant qu'élément porteur (130) en forme de disque, notamment en tant qu'élément porteur (130) en forme de disque circulaire et de manière particulièrement préférée en tant qu'élément porteur (130) en forme d'anneau de cercle, la substance chimique (134) de test étant appliquée en tant que revêtement sur l'élément porteur (130), le revêtement mettant à disposition des zones de surface de test (136) pour les outils d'analyse (112).

11. Magasin d'analyse (110) comprenant une pluralité d'outils d'analyse (112) pour détecter la présence d'au moins un analyte dans un échantillon, les outils d'analyse (112) comportant chacun :
- au moins un boîtier (120) avec au moins une partie de boîtier (114) et
- au moins un élément de test (122) avec au moins une substance chimique (134) de test,
les outils d'analyse (112) étant réceptionnés dans un boîtier formant un magasin (118) commun, les boîtiers (120) des outils d'analyse (112) étant des composants du boîtier formant un magasin (118), les parties de boîtier (114) étant fabriquées au moyen d'un procédé de façonnage et étant reliées à l'élément de test respectif (122) au moyen du procédé de façonnage, les parties de boîtier (114) des outils d'analyse (112) étant fabriquées simultanément et étant reliées les unes aux autres simultanément au cours du procédé de façonnage.

12. Magasin d'analyse (110) selon la revendication précédente, les outils d'analyse (112) comportant chacun par ailleurs au moins un élément de lancette pour générer l'échantillon, notamment au moins un microlysimètre.

13. Magasin d'analyse (110) selon l'une quelconque des revendications 11 ou 12, les boîtiers (120) comportant chacun au moins un regard (146) accessible à partir d'une face extérieure du boîtier (120), au moins une modification des propriétés de l'élément de test respectif (122), notamment au moins un variation de couleur et/ou au moins une modification d'au moins une propriété optique étant détectable par l'extérieur, à travers le regard (146).

14. Magasin d'analyse (110) selon la revendication précédente, le regard (146) étant conçu de telle sorte qu'au moins une surface de zone de test (136) de l'élément de test respectif (122) puisse être observée à travers au moins un élément porteur (130) optiquement au moins en partie transparent de l'élément de test respectif (122).
